(19) Europäisches Patentamt
European Patent Office
Office européen des brevets

(11) Publication number: **0 484 614 B1**

# (12) EUROPEAN PATENT SPECIFICATION

(45) Date of publication of patent specification: **24.05.95**

(51) Int. Cl.⁶: **C07D 207/34**, C07D 207/42, C07D 405/06, C07D 401/06, A01N 43/36

(21) Application number: **91103923.8**

(22) Date of filing: **14.03.91**

## (54) N-acylated arylpyrroles useful as insecticidal, acaricidal, nematicidal and molluscicidal agents.

(30) Priority: **11.05.90 US 522299**

(43) Date of publication of application:
**13.05.92 Bulletin 92/20**

(45) Publication of the grant of the patent:
**24.05.95 Bulletin 95/21**

(84) Designated Contracting States:
**AT BE CH DE DK ES FR GB GR IT LI LU NL SE**

(56) References cited:
**EP-A- 0 347 488**
**EP-A- 0 372 263**
**EP-A- 0 426 948**

(73) Proprietor: **AMERICAN CYANAMID COMPANY**
**One Cyanamid Plaza**
**Wayne, NJ 07470-8426 (US)**

(72) Inventor: **Addor, Roger Williams**
**P.O. Box 347A RR1 Woosamonsa Road**
**Pennington,**
**New Jersey 08534 (US)**
Inventor: **Donovan, Stephen Francis**
**408 Lenape Lane**
**Yardley,**
**Pennsylvania 19/67 (US)**
Inventor: **Diehl, Robert Eugene**
**17 Clementon Way**
**Lawrenceville,**
**New Jersey 08638 (US)**
Inventor: **Kremer, Kenneth Alfred Martin**
**B-15 CArver Place**
**Lawrenceville,**
**New Jersey 08647 (US)**

(74) Representative: **Wächtershäuser, Günter, Prof. Dr.**
**Patentanwalt,**
**Tal 29**
**D-80331 München (DE)**

**EP 0 484 614 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

## DESCRIPTION OF THE INVENTION

This invention relates to new N-acylated arylpyrroles depicted by formula I:

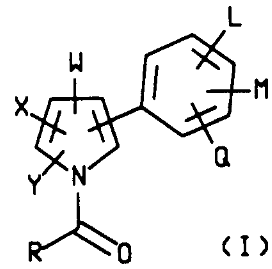

(I)

wherein

| | |
|---|---|
| X | is H, F, Cl, Br, I, or $CF_3$; |
| Y | is F, Cl, Br, I, or $CF_3$; |
| W | is CN, or $NO_2$; |
| L | is H, F, Cl or Br; and |
| M and Q | are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$, $R_2CO$ or $NR_3R_4$; |

and when M and Q are attached to adjacent carbons in the phenyl ring and taken with the carbon atoms to which they are attached, they may form a ring in which MQ represents the structure:

$-OCH_2O-$, $-OCF_2O-$ or

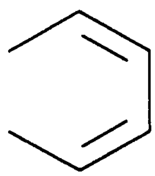

;

| | |
|---|---|
| Z | is S(O)n or O; |
| $R_1$ | is H, F, $CHF_2$, CHFCl, or $CF_3$; |
| $R_2$ | is $C_1$-$C_3$ alkyl, $C_1$-$C_3$ alkoxy, or $NR_3R_4$; |
| $R_3$ | is H or $C_1$-$C_3$ alkyl; |
| $R_4$ | is H, $C_1$-$C_3$ alkyl, or $R_5CO$; |
| $R_5$ | is H or $C_1$-$C_3$ alkyl; and |
| n | is an integer of 0, 1 or 2; and |
| R | is $C_1$-$C_6$ alkyl optionally substituted with one to three halogen atoms, |

one hydroxy,

one cyano,

one or two $C_1$-$C_4$ alkoxy groups optionally substituted with one to three halogen atoms,

one $C_1$-$C_4$ alkylthio,

one phenyl group optionally substituted with one to three halogen atoms, one to three $C_1$-$C_4$ alkyl groups or one to three $C_1$-$C_4$ alkoxy groups,

one phenoxy group optionally substituted with one to three halogen atoms, one to three $C_1$-$C_4$ alkyl groups or one to three $C_1$-$C_4$ alkoxy groups,

one benzyloxy group optionally substituted on the phenyl ring with one to three halogen atoms, one to three $C_1$-$C_4$ alkyl groups or one to three $C_1$-$C_4$ alkoxy groups,

one $C_1$-$C_6$ alkyl carbonyloxy group optionally substituted with one to three halogen atoms,

one $C_2$-$C_6$ alkenylcarbonyloxy group optionally substituted with one to three halogen atoms,

one phenylcarbonyloxy group optionally substituted with one to three halogen atoms, one to three $C_1$-$C_4$ alkyl groups or one to three $C_1$-$C_4$ alkoxy groups,

one $C_1$-$C_6$ alkoxycarbonyl group optionally substituted with one to three halogen atoms, or one to three $C_1$-$C_4$ alkoxy groups, or

one benzyloxycarbonyl group optionally substituted on the phenyl ring with one to three halogen atoms, one to three $C_1$-$C_4$ alkyl groups or one to three $C_1$-$C_4$ alkoxy groups,

$C_2$-$C_6$ alkenyl optionally substituted with one to three halogen atoms or one phenyl group,

$C_3$-$C_6$ alkynyl optionally substituted with one to three halogen atoms or one phenyl group,

$C_3$-$C_6$ cycloalkyl,

phenyl optionally substituted with one to three halogen atoms, one to three $C_1$-$C_4$ alkyl groups, one $C_5$-$C_{12}$ alkyl group, one to two $C_1$-$C_4$ alkoxy groups, or one phenoxy, $C_1$-$C_4$ alkylthio, trialkylsilyl, $C_1$-$C_4$ alkylsulfinyl, $C_1$-$C_4$ alkylsulfonyl, carbo-$C_1$-$C_4$-alkoxy, carboxy, $CF_3$, CN, $NO_2$, di($C_1$-$C_4$ alkyl)amino, or $C_1$-$C_4$ alkanoylamino,

phenoxy optionally substituted with one to three halogen atoms, one or two $C_1$-$C_4$ alkyl groups, one or two $C_1$-$C_4$ alkoxy groups, trialkylsilyl, $CF_3$, CN, $NO_2$, or di($C_1$-$C_4$ alkyl)amino groups, or $C_1$-$C_4$ alkanoylamino,

1-naphthyl or 2-naphthyl,

2-, 3-, or 4-pyridyl optionally substituted with one to three halogen atoms, a heteroaromatic 5-membered ring containing an oxygen, nitrogen, or a sulfur atom, and optionally substituted with one to three halogen atoms,

$C_1$-$C_6$ alkoxy group optionally substituted with one to three halogen atoms, or

$C_2$-$C_6$ alkenyloxy group optionally substituted with one to three halogen atoms.

A preferred group of compounds of this invention are depicted by the structural formula II, which may be illustrated as follows:

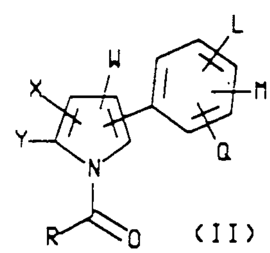

(II)

wherein L, M, Q, W, X, Y and R are as described for formula I above.

Another preferred group of compounds of this invention have the structure of formula II, where W is CN; X is H, F, Cl, Br or $CF_3$; Y is H, Cl, Br or $CF_3$; L is H, Cl, F or Br; M and Q are each independently, H, halogen or $CF_3$; and R is

phenyl optionally substituted with one to three halogen atoms, one to three $C_1$-$C_4$ alkyl groups, one to two $C_1$-$C_4$ alkoxy groups, or one phenoxy, $C_1$-$C_4$ alkylthio, trialkylsilyl, $C_1$-$C_4$ alkylsulfinyl, $C_1$-$C_4$ alkylsulfonyl, carbo-$C_1$-$C_4$-alkoxy, carboxy, $CF_3$, CN, $NO_2$, di($C_1$-$C_4$ alkyl)amino, or $C_1$-$C_4$ alkanoylamino,

1-naphthyl or 2-naphthyl,

2-, 3-, or 4-pyridyl optionally substituted with one to three halogen atoms, or a heteroaromatic 5-membered ring containing an oxygen, nitrogen, or a sulfur atom, and optionally substituted with one to three halogen atoms.

Preparation of the N-acylated arylpyrroles of the invention generally involves the acylation of an arylpyrrole having the formula III structure:

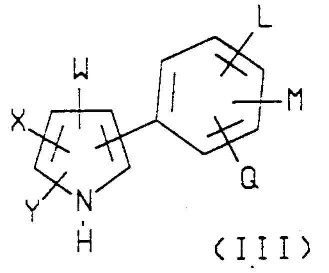

(III)

wherein L, M, Q, W, X and Y are as described for formula I above.

Acylation of the formula III arylpyrrole can be achieved by the reaction of said formula III arylpyrrole with an alkali metal hydride or alkali metal t-butoxide and an acylating agent having the structure: RCOCl wherein R is as described above.

In the reaction the alkali metal hydride, preferrably sodium hydride, or alkali metal t-butoxide is generally dispersed in an anhydrous organic solvent such as dry tetrahydrofuran, dimethoxyethane, dimethylformamide, dimethylsulfoxide, or the like, and the thus formed mixture then heated to refluxing temperature. The reaction mixture is then cooled, generally to between 20 °C and 30 °C, and the acylating agent, RCOCl, wherein R is as described above, added to said mixture. Thereafter, the thus prepared mixture is heated to refluxing temperature until the N-acylated arylpyrrole is formed. The reactions are preferably conducted under a blanket of inert gas such as nitrogen or argon. The reactions may be illustrated as follows:

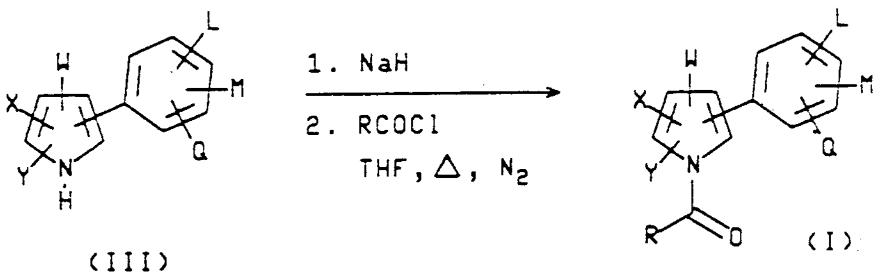


(III) (I)

The formula I N-acylated arylpyrroles of this invention are effective for the control of a wide variety of destructive plant pests that ravage all types of vegetation including field crops, forage crops, cotton, cucurbits, cereal grains, ornamentals, cole crops, shrubs and the like.

Accordingly, the present invention provides a method for controlling insects, acarina, nematodes, fungi or mollusks which comprises contacting said insects, acarina, nematodes, fungi or mollusks, their breeding grounds, food supply or habitat with an insecticidally, acaricidally, nematicidally, fungicidally or molluscicidally effective amount of an N-acylated arylpyrroleas described in claim 1.

Among these destructive pests are the insects and acarina that feed upon the foliage, stems, fruits, flowers and sap of plants. These insects and acarina are generally of the orders: Coleoptera, Diptera, Thysanoptera, Homoptera, Hymenoptera, Lepidoptera and Orthoptera. Other pests that are destructive to plant life, but are readily controlled with the formula I N-acylated arylpyrroles of this invention, are soil-borne pests such as nematodes, rootworms, wireworms and the like which attack and destroy the root systems of plants and mollusks, particularly of the class gastropada, which includes slugs, cowries, limpets and snails, that are voracious feeders with an appetite for seedling plants, especially ornamentals. It is further found that the N-acylated arylpyrroles of this invention have the added advantage that they may also be used for the control of fungal organisms and the protection of plants against the ravages of fungal diseases.

To achieve control of the above-said pests and/or provide protection of plants from attack thereby, it has been found that the N-acylated arylpyrroles of this invention can be prepared in the form of aqueous or liquid formulations and applied to the plants and/or soil in which they are growing as an aqueous or liquid spray or drench containing from about 10 ppm to about 10,000 ppm and preferably about 50 ppm to 4000 ppm of the formula I N-acylated arylpyrrole. These liquid compositions are usually applied in sufficient amount to provide about 0.1 kg/ha to about 4.0 kg/ha of the active ingredient to the locus of treatment.

They may also be prepared as solid formulations such as compacted granules, dusts, dust concentrates and bait formulations which may be applied to the soil in which plants are to be protected, or, in the case of dusts or dust concentrates they may be applied to the foliage of plants.

The aqueous or liquid compositions useful in the practice of this invention may be initially formulated as a solid or liquid concentrate which is dispersed in water or other inexpensive liquid diluent, generally at the locus of treatment, for spray or drench application.

The concentrates useful for preparation of sprays or drenches may take the form of a wettable powder, emulsifiable concentrate, aqueous flowable or water dispersible granular formulation.

A typical suspension concentrate formulation may be prepared by grinding together about 5% to 25% by weight of a formula I N-acylated arylpyrrole, about 3% to 20% by weight of an anionic surfactant such as dodecyl benzene sulfonic acid, about 1% to 5% by weight of a nonionic surfactant such as an ethylene oxide block copolymer having about 8 to 11 mols of ethoxylation, about 1% to 5% by weight of an alkylphenol polyethylene oxide condensate with 9 to 10 mols of ethoxylation and q.s. to 100% with a petroleum aromatic solvent.

A preferred group of ethylene oxide/propylene oxide block copolymers for use in the compositions of this invention are butyl-omega-hydroxypoly(oxypropylene)-block polymer with poly(oxyethylene) having an average molecular weight in a range of 2,400 to 3,500, with alpha-butyl-omega-hydroxy-ethylene oxide-propylene oxide block copolymers having an HLB of 12 and a viscosity at 25°C of 2000 CPS, (TOXIMUL® 8320, Stepan Chemical Co.) being a most preferred member of this class of emulsifiers.

Preferred alkylphenol polyethylene oxide condensates for use in the compositions of the invention are the nonylphenol ethoxylates, with nonylphenol ethoxylate (9 to 10 mols of ethylene oxide) (FLA MO® 9N, DeSoto, Inc. Sellers Chemical Div.) being a most preferred member of this class of emulsifiers.

Among the preferred petroleum aromatic solvents useful in the preparation of suspension concentrates containing the formula I N-acylated arylpyrroles of the present invention are:

1) aromatic hydrocarbon mixture ($C_9$ to $C_{12}$ aromatics, distillation range 183-210°C) (AROMATIC® 150, Exxon)

2) aromatic hydrocarbon mixture ($C_8$ to $C_9$ aromatics, distillation range 155-173°C) (AROMATIC® 100, Exxon)

3) aromatic hydrocarbon mixture ($C_{10}$ to $C_{13}$ aromatics, distillation range 226-279°C) (AROMATIC® 200)

4) aromatic hydrocarbon mixture ($C_8$ to $C_9$ aromatics, bp 148.9°C) (TENNECO® T500/100) aromatic hydrocarbon mixture (TENNECO® T400) hydrocarbon mixture, (distillation range 177-277°C) (HAN® Exxon)

5) aromatic hydrocarbon mixture (distillation range 210-288°C) (PANASOL® AN-3N, Amoco)

6) aromatic hydrocarbon mixture (distillation range 179-216°C) (Shell CYCLO SOL® 63)

Flowable formulations can be prepared by admixing about 5% to 50% and preferably about 10% to 25% by weight of the formula I N-acylated arylpyrrole with about 2% to 3% by weight of a naphthalene sulfonic condensate, about 0.1% to 0.5% by weight of a nonionic nonylphenoxy polyethoxy ethanol, about 0.1% to 0.5% of xanthum gum, about 0.1% to 0.5% of a swelling clay such as bentonite, about 5% to 10% by weight of propylene glycol, about 0.1% to 0.5% by weight of a silicone antifoam agent, about 0.1% to 0.3% by weight of an aqueous dipropylene glycol solution of 1,2-benzisothiazolin-3-one (preservative) and q.s. to 100% with water.

A wettable powder can be prepared by grinding together about 5% to 25% by weight of the formula I N-acylated arylpyrrole about 3% to 15% by weight of an anionic surfactant, such as dodecylbenzene sulfonic acid, about 3% to 10% of a nonionic ethylene oxide block copolymer, about 1% to 3% by weight of a nonylphenol ethoxylate having 8 to 11 mols of ethoxylation and about 47% to 88% by weight of an inert solid diluent such as montmorillonite, attapulgite, diatomaceous earth, talc or the like.

In addition to the suspension concentrates, aqueous flowables and wettable powders described hereinabove, other formulations such as water dispersible granules, emulsifiable concentrates and compacted granular formulations may also be prepared and used to protect plants from attack by the pests mentioned above.

From the viewpoint of utility and practical application, several advantages may accrue from the various acylations of insecticidal pyrroles described herein. In one instance, such N-derivatization leads to improved product solubility in organic solvents thus affording a greater ease in formulating the product for application. In those cases where the parent pyrrole exhibits little or no phytotoxicity to agronomic crops, little attention need be paid to the hydrolytic stability of the resulting derivative as long as the stability allows formulation in a non-aqueous medium and a tank-mix stability sufficiently long to allow application. Such an instance is found, for example, with compound A. In this case, the compound is non-injurious to plants. However,

because of poor solubility properties, A, illustrated below, is difficult to formulate. When, however, A is acetylated to give B, solubility in organic solvents is improved allowing the formulations chemist a greater latitude in designing a suitable vehicle for applying the product. Such formulations may be dispersed in water as is the usual practice and applied forthwith even though the acetylated pyrrole B will largely revert to the parent A if left for several days in a homogenous mixture with water and a miscible solvent such as acetone.

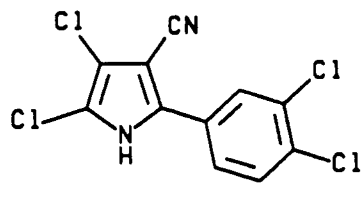

A

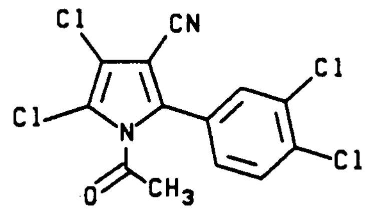

B

If a pyrrole such as A is benzoylated to give a product C, solubility properties are again improved. In addition, however, whereas both A and C have comparable insecticidal activity against the larval stages of the southern armyworm and the tobacco budworm, C is also found to be effective against the two-spotted spider mite at a concentration of 100 ppm. This contrasts to A which is without activity even at 300 ppm. Benzoylated pyrroles such as C are also found to have much greater hydrolytic stability than those derived from aliphatic carboxylic acids. This distinction is of little concern where the parent pyrrole is non-phytotoxic and a small amount of the parent generated by hydrolysis would not harm the plants to be protected from insects. However, in a number of cases we have found pyrroles which are highly active against insects but injurious to plants they are designed to protect. An example is compound D, a highly effective broad-spectrum insecticide which cannot be used because of severe plant damage. Since even small concentrations of this pyrrole in a formulation are unacceptable, the derivative must be non-phytotoxic and hydrolytically stable. In this case, the benzoylated product E provides a plant-safe product. Danger of in-situ hydrolytic generation of the phytoxic parent is very slight since E is found to have a half-life in a homogenous water-acetonitrile mixture measured in years. In this case there is also the added bonus of reduced mammalian toxicity, that for the parent D being 31 mg/kg vs. 54 mg/kg for E as the dose required to kill 50% of orally dosed mice.

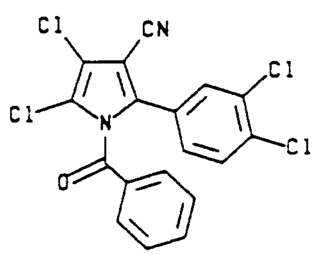

C

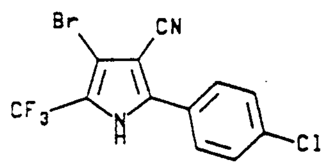

D

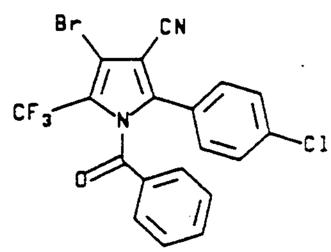

E

These and other advantages of the invention may become more obvious from the examples setforth below. These examples are provided simply for illustrative purposes.

EXAMPLE 1

**Preparation of 1-Benzoyl-4-bromo-2-chloro-5-(p-chlorophenyl)pyrrole-3-carbonitrile**

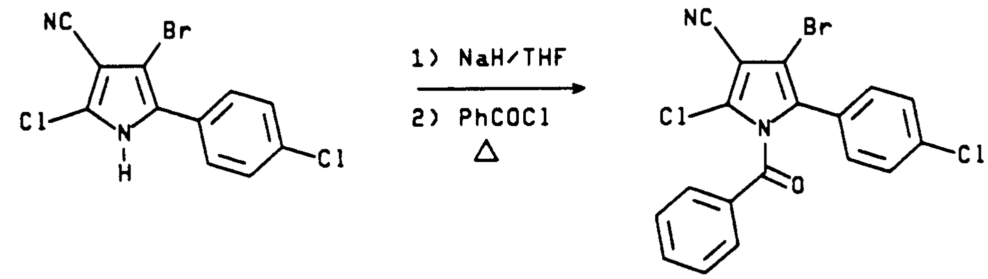


To a suspension of sodium hydride (0.21 g of a 60% dispersion, 5.3 mmol) in 50 mL of dry tetrahydrofuran in a 100 mL single neck round bottom flask fitted with a condenser and a nitrogen inlet is added portion-wise 4-bromo-2-chloro-5-(p-chlorophenyl)pyrrole-3-carbonitrile (1.0 g, 3.2 mmol). The reaction is stirred for 15 minutes at room temperature before the pipette addition of hexamethylphosphoramide (HMPA) (3 mL) followed immediately by benzoyl chloride (0.75 g, 5.4 mmol). Thereafter the reaction mixture is heated to reflux for 17 hours and allowed to cool. Rotary evaporation yields a crude semi-solid to which is added 15 mL of diethyl ether and 15 mL of water. Vigorous stirring over a period of 20 minutes followed by vacuum filtration results in the isolation of the product as a yellow solid (1.0 g, 2.4 mmol, 75%), mp 179-181 °C.

EXAMPLE 2

**Preparation of 1-Benzoyl-4-bromo-2-(p-chlorophenyl)-5-(trifluoromethyl)pyrrole-3-carbonitrile**

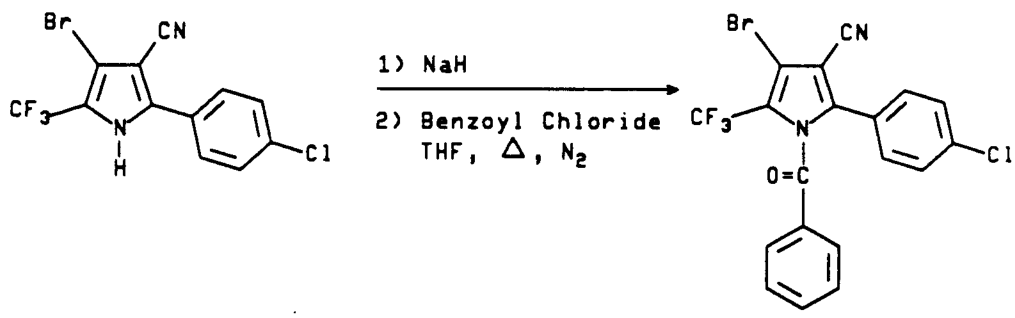


To a 250 ml round bottom flask, equipped with a magnetic stirrer and a condenser with a nitrogen adapter, is added 0.60 g. NaH (60%). The NaH is washed with ca. 50 ml hexanes, the hexanes are decanted, and replaced with 75 ml tetrahydrofuran (THF). There is some bubbling upon the addition of the THF, therefore the THF is not quite anhydrous. The flask is cooled in an ice-water bath and 2.04 g of 4-bromo-2-(p-chlorophenyl)-5-(trifluoromethyl)pyrrole-3-carbonitrile is added in portions with the evolution of hydrogen. The reaction mixture is refluxed for 20 minutes, cooled to ca. room temperature and then 2.3 ml benzoyl chloride is added and the reaction mixture is refluxed overnight. The reaction is cooled and poured

into ca. 100 ml of cold water and extracted with 100 ml ether. The ether extract is washed with 100 ml water, 100 ml saturated $NaHCO_3$, dried over $K_2CO_3$, filtered, and concentrated on a rotary evaporator under reduced pressure. The excess benzoyl chloride is removed by distillation with a kugelrohr apparatus at 100 °C at -5 mm Hg. Then the residue is refluxed with 100 ml hexanes, and filtered warm to remove most of the unreacted 4-bromo-2-(p-chlorophenyl)-5-(trifluoromethyl)pyrrole-3-carbonitrile. The reaction product is then chromatographed on a "flash" silica gel column ca. 1" by 12" eluted by 10% ethyl acetate and 90% hexanes. Then 100 ml fractions are collected and fractions #3 and #4 are combined and concentrated on a rotary evaporator under reduced pressure. Then 50 ml hexanes are added, the mixture is refluxed to bring the product into solution and the solution allowed to slowly crystallize overnight. After filtration 1.58 grams of white crystals of 1-benzoyl-4-bromo-2-(p-chlorophenyl)-5-(trifluoromethyl)pyrrole-3-carbonitrile are isolated. Melting point 105-107 °C.

EXAMPLE 3

**Preparation of 4-Bromo-2-(p-chlorophenyl)-1-pivaloyl-5-(trifluoromethyl)pyrrole-3-carbonitrile**

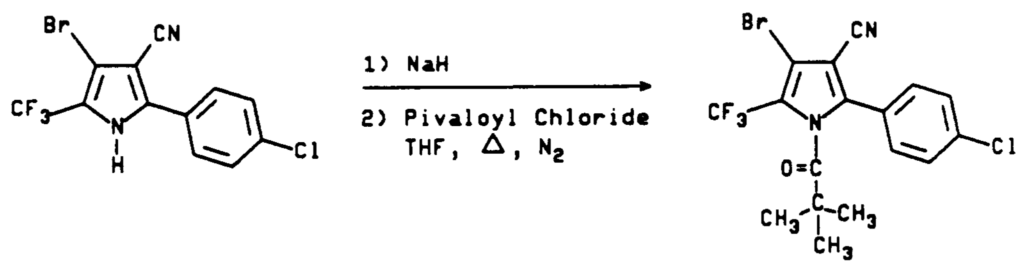


To a 250 ml round bottom flask, equipped with a magnetic stirrer and a condenser with a nitrogen adapter, is added 1.20 g. NaH (60%). The NaH is washed with ca. 50 ml hexanes, the hexanes are then decanted, and replaced with 75 ml of tetrahydrofuran (THF). There is some bubbling upon the addition of the THF, therefore the THF is not quite anhydrous. The flask is cooled in an ice-water bath and 4.08 g 4-bromo-2-(p-chlorophenyl)-5-(trifluoromethyl)pyrrole-3-carbonitrile is added in portions with the evolution of hydrogen. The reaction mixture is refluxed for 20 minutes, is cooled to ca. room temperature and then 5.0 ml of pivaloyl chloride is added and the reaction mixture is refluxed overnight. The progress of the reaction is assessed by a HPLC of an aliquot. The HPLC indicates that the reaction has progessed about 20% to completion. Then 2 ml hexamethylphosphoramide (HMPA) is added to speed up the reaction, and the reaction is again refluxed overnight. The reaction mixture is cooled and poured into ca. 100 ml of cold water and extracted with 100 ml ether and 100 ml hexanes. The organic layer is washed three times with 100 ml water, then 100 ml sat. NaCl, dried over $K_2CO_3$, filtered, and concentrated on a rotary evaporator under reduced pressure. Then to the residue is added 100 ml of hexanes. The mixture is refluxed, cooled, and then filtered to give 3.54 grams of a tan powder. The powder is crystallized twice from 50 ml of methylcyclohexanes to give 2.75 grams of 4-bromo-2-(p-chlorophenyl)-1-pivaloyl-5-(trifluoromethyl)pyrrole-3-carbonitrile as tan needles, M.P. = 177-183 °C.

Following the above procedure, but substituting the appropriate arylpyrrole for 4-bromo-2-(p-chlorophenyl)-5-(trifluoromethyl)pyrrole-3-carbonitrile and the appropriate acylhalide RCOCl for pivaloyl chloride yields the following compounds.

4-bromo-2-(p-chlorophenyl)-1-methacryloyl-5-(trifluoromethyl)pyrrole-3-carbonitrile M.P. 132.5-134 °C;
4-bromo-2-(p-chlorophenyl)-1-o-toluoyl-5-(trifluoromethyl)pyrrole-3-carbonitrile M.P. 131.5-134.5 °C;
4-bromo-1-(m-chlorobenzoyl)-1-(p-chlorophenyl)-5-(trifluoromethyl)pyrrole-3-carbonitrile M.P. 88-90 °C;
4-bromo-2-(p-chlorophenyl)-1-(2-furoyl)-5-(trifluoromethyl)pyrrole-3-carbonitrile M.P. 152-156 °C;
4-bromo-2-(p-chlorophenyl)-1-p-toluoyl-5-(trifluoromethyl)pyrrole-3-carbonitrile M.P. 113-116.5 °C;
4-bromo-2-(p-chlorophenyl)-5-(trifluoromethyl)-1-(α,α,α-trifluoro-p-toluoyl)pyrrole-3-carbonitrile; M.P. 110-118 °C.

4-bromo-2-(p-chlorophenyl)-1-(p-nitrobenzoyl)-5-(trifluoromethyl)pyrrole-3-carbonitrile M.P. 128-132 °C;
phenyl 3-bromo-5-(p-chlorophenyl)-4-cyano-2-(trifluoromethyl)pyrrole-1-carboxylate M.P. 116-120 °C;
4-bromo-1-(p-chlorobenzoyl)-2-(p-chlorophenyl)-5-(trifluoromethyl)pyrrole-3-carbonitrile M.P. 115-117 °C;
4-bromo-2-(p-chlorophenyl)-1-(cyclohexylcarbonyl)-5-(trifluoromethyl)pyrrole-3-carbonitrile M.P. 141-142 °C;
4-bromo-2-(p-chlorophenyl)-1-pivaloyl-5-(trifluoromethyl)pyrrole-3-carbonitrile M.P. 177-183 °C;
1-acetyl-4,5-dichloro-2-(3,4-dichlorophenyl)pyrrole-3-carbonitrile M.P.dec. 150 °C;
phenyl 2,3-dibromo-4-cyano-5-($\alpha,\alpha,\alpha$-trifluoro-p-tolyl)pyrrole-3-carboxylate M.P. 148-149 °C;
4-bromo-2-(p-chlorophenyl)-1-(1-naphthoyl)-5-(trifluoromethyl)pyrrole-3-carbonitrile M.P. 101-108 °C;
4-bromo-2-(p-chlorophenyl)-1-(m-fluorobenzoyl)-5-(trifluoromethyl)pyrrole-3-carbonitrile M.P. 111-118 °C;
4-bromo-2-(p-chlorophenyl)-1-(3,4-dichlorobenzoyl)-5-(trifluoromethyl)pyrrole-3-carbonitrile M.P. 135-144 °C;
1-benzoyl-4,5-dichloro-2-(3,4-dichlorophenyl)pyrrole-3-carbonitrile M.P. 141-144 °C;
4-bromo-1-(p-tert-butylbenzoyl)-2-(p-chlorophenyl)-5-(trifluoromethyl)pyrrole-3-carbonitrile M.P. 113-115.5 °C; or
1-benzoyl-4-bromo-2-(p-bromophenyl)-5-(trifluoromethyl)pyrrole-3-carbonitrile M.P. 112-117 °C.

## EXAMPLE 4

### Insecticidal and acaricidal evaluations of N-acylated arylpyrroles

In these tests evaluations are performed using technical material dissolved in 50/50 acetone water mixtures. All concentrations reported herein are in terms of active ingredient. All tests are conducted in a laboratory maintained at about 27 °C. The rating system employed is as follows:

| Rating System | |
|---|---|
| o = no effect | 5 = 56-65% kill |
| 1 = 10-25% kill | 6 = 66-75% kill |
| 2 = 26-35% kill | 7 = 76-85% kill |
| 3 = 36-45% kill | 8 = 86-99% kill |
| 4 = 46-55% kill | 9 = 100% kill |

Where two or more tests are conducted using the same compound, the average of test results is reported.

The test species of insects and acarids used in the present evaluations along with specific test procedures are described below.

### Heliothis virescens, 3rd instar tobacco budworm

Cotton cotyledons are dipped in the test solution and allowed to dry in a hood. When dry, each is cut into quarters and ten sections placed individually in 30 ml plastic medicine cups containing a 5-7 mm long piece of damp dental wick. One third-instar caterpillar is added to each cup and a cardboard lid placed on the cup. Treatments are maintained for 3 days before mortality counts and estimates of reduction in feeding damage are made.

### Spodoptera eridania, 3rd instar larvae, southern armyworm

A Sieva lima bean leaf expanded to 7-8 cm in length is dipped in the test solution with agitation for 3 seconds and placed in a hood to dry. The leaf is then placed in a 100x10 mm petri dish containing a damp filter paper on the bottom and ten 3rd instar caterpillars. The dish is maintained for 5 days before observations are made of mortality, reduced feeding, or any interference with normal moulting.

### Spodontera eridania, 7-day residual

The plants treated in the above Test are maintained under high intensity lamps in the greenhouse for 7 days. These lamps duplicate the effects of a bright sunny day in June in New Jersey and are kept on for 14

hour day length. After 7 days, the foliage is sampled and assayed as in the above-said Test.

Diabrotic undecimpunctata howardi, 3rd instar southern corn rootworm

One cc of fine talc is placed in a 30 ml wide-mouth screw-top glass jar. One ml of the appropriate acetone solution is pipetted onto the talc so as to provide 1.25 and 0.25 mg of active ingredient per jar. The jars are set under a gentle air flow until the acetone is evaporated. The dried talc is loosened, 1 cc of millet seed is added to serve as food for the insects and 25 ml of moist soil is added to each jar. The jar is capped and the contents thoroughly mixed on a Vortex Mixer. Following this, ten 3rd instar rootworms are added to each jar and the jars are loosely capped to allow air exchange for the larvae. The treatments are held for 6 days before mortality counts are made. Missing larvae are presumed dead, since they decompose rapidly and can not be found. The concentrations used in this test correspond approximately to 50 and 10 kg/ha, respectively.

Tetranychus urticae (P-resistant strain), 2-spotted spider mite

Sieva lima bean plants with primary leaves expanded to 7-8 cm are selected and cut back to one plant per pot. A small piece is cut from a leaf taken from the main colony and placed on each leaf of the test plants. This is done about 2 hours before treatment to allow the mites to move over to the test plant and to lay eggs. The size of the cut piece is varied to obtain about 100 mites per leaf. At the time of the treatment, the piece of leaf used to transfer the mites is removed and discarded. The mite-infested plants are dipped in the test solution for 3 seconds with agitation and set in the hood to dry. Plants are kept for 2 days before estimates of adult kill are made using the first leaf. The second leaf is kept on the plant for another 5 days before observations are made of the kill of eggs and/or newly emerged nymphs.

Empoasca abrupta, adults, western potato leafhopper

A Sieva lima bean leaf about 5 cm long is dipped in the test solution for 3 seconds with agitation and placed in a hood to dry. The leaf is placed in a 100x10 mm petri dish containing a moist filter paper on the bottom. About 10 adult leafhoppers are added to each dish and the treatments are kept for 3 days before mortality counts are made.

Blattella germanica, residue test, adult male German cockroach

One ml of a 1000 ppm acetone solution of the test material is pipetted slowly over the bottom of a 150x15 mm petri dish so as to give as uniform coverage as possible. After the deposit has dried, 10 adult male cockroaches are placed in each dish and the lid is added. Mortality counts are made after 3 days.

In these evaluations X = Test was not completed; - = Test could not be conducted; a blank means Test was not conducted and * = Data not yet reported.

Data obtained are reported in Table I below.

Compounds evaluated in these tests include:

| Compound # | Compound Name |
|---|---|
| 1 | 1-Benzoyl-4-bromo-2-(p-chlorophenyl)-5-(trifluoromethyl)pyrrole-3-carbonitrile, M.P. 105-107°C; |
| 2 | 4-bromo-2-(p-chlorophenyl)-1-methacryloyl-5-(trifluoromethyl)pyrrole-3-carbonitrile M.P. 132.5-134°C; |
| 3 | 4-bromo-2-(p-chlorophenyl)-1-o-toluoyl-5-(trifluoromethyl)pyrrole-3-carbonitrile M.P. 131.5-134.5°C; |
| 4 | 4-bromo-1-(m-chlorobenzoyl)-1-(p-chlorophenyl)-5-(trifluoromethyl)pyrrole-3-carbonitrile M.P. 88-90°C; |
| 5 | 4-bromo-2-(p-chlorophenyl)-1-(2-furoyl)-5-(trifluoromethyl)pyrrole-3-carboni- |

trile M.P. 152-156°C;

6 4-bromo-2-(p-chlorophenyl)-1-p-toluoyl-5-(trifluoromethyl)pyrrole-3-carbonitrile M.P. 113-116.5°C;

7 4-bromo-2-(p-chlorophenyl)-5-(trifluoromethyl)-1-(α,α,-α-trifluoro-p-toluoyl)-pyrrole-3-carbonitrile; M.P. 110-118°C.

8 4-bromo-2-(p-chlorophenyl)-1-(p-nitrobenzoyl)-5-(trifluoromethyl)pyrrole-3-carbonitrile M.P. 128-132°C;

9 Phenyl 3-bromo-5-(p-chlorophenyl)-4-cyano-2-(trifluoromethyl)pyrrole-1-carboxylate M.P. 116-120°C;

10 4-bromo-1-(p-chlorobenzoyl)-2-(p-chlorophenyl)-5-(trifluoromethyl)pyrrole-3-carbonitrile M.P. 115-117°C;

11 4-bromo-2-(p-chlorophenyl)-1-(cyclohexylcarbonyl)-5-(trifluoromethyl)pyrrole-3-carbonitrile M.P. 141-142°C;

12 1-benzoyl-4-bromo-2-chloro-5-(p-chlorophenyl)pyrrole-3-carbonitrile M.P. 179-181°C;

13 4-bromo-2-(p-chlorophenyl)-1-pivaloyl-5-(trifluoromethyl)pyrrole-3-carbonitrile M.P. 177-183°C;

14 1-acetyl-4,5-dichloro-2-(3,4-dichlorophenyl)pyrrole-3-carbonitrile M.P.dec. 150°C;

15 Phenyl 2,3-dibromo-4-cyano-5-(α,α,α-trifluoro-p-tolyl)pyrrole-3-carboxylate M.P. 148-149°C;

16 4-bromo-2-(p-chlorophenyl)-1-(1-naphthoyl)-5-(trifluoromethyl)pyrrole-3-carbonitrile M.P. 101-108°C;

17 4-bromo-2-(p-chlorophenyl)-1-(m-fluoro-

| | |
|---|---|
| | benzoyl)-5-(trifluoromethyl)pyrrole-3-carbonitrile M.P. 111-118°C; |
| 18 | 4-bromo-2-(p-chlorophenyl)-1-(3,4-dichlorobenzoyl)-5-(trifluoromethyl)pyrrole-3-carbonitrile M.P. 135-144°C; |
| 19 | 4-bromo-1-(p-tert-butylbenzoyl)-2-(p-chlorophenyl)-5-(trifluoromethyl)pyrrole-3-carbonitrile M.P. 113-115.5°C; or |
| 20 | 1-benzoyl-4-bromo-2-(p-bromophenyl)-5-(trifluoromethyl)pyrrole-3-carbonitrile M.P. 112-117°C. |

Table I

**Insecticidal and Acaricidal Evaluation of N-acylated Arylpyrroles**

| Compound | Budworm | Armyworm | | S. Corn | OP Resistant Mites | | | Leafhopper | Roach |
|---|---|---|---|---|---|---|---|---|---|
| Number | 3rd | Day 3 | Day 5 | Rootworm | Adult | Egg | Nymph | Cont. | Residual |
| | ppm | ppm | ppm | ppm | ppm | ppm | ppm | ppm | ppm |
| | 1 | 1 R | 1 R | | | | | | 1 |
| | 0 1 | 0 1 E | 0 1 E | | 3 1 | 3 1 | 3 1 | 1 | 0 1 |
| | 0 0 1 | 0 0 1 S | 0 0 1 S | 5 1 | 0 0 1 | 0 0 1 | 0 0 1 | 0 1 | 0 0 1 |
| | 0 0 0 | 0 0 0 i | 0 0 0 i | 0 0 1 | 0 0 0 | 0 0 0 | 0 0 0 | 0 0 1 | 0 0 0 |
| 1 | 9 9 8 | 9 9 9 9 | 9 9 9 9 | 9 5 4 | 9 9 9 | 0 9 9 | 9 - - | 9 9 6 | 9 9 7 |
| 2 | 9 9 9 | 9 9 9 9 | 9 9 9 9 | 9 5 - | 9 9 9 | 9 9 9 | x - - | 9 9 0 | 9 9 9 |
| 3 | 0 0 1 | 9 9 9 9 | 9 9 9 9 | 9 9 8 | 9 9 9 | 9 9 8 | x - 9 | 9 9 0 | 9 9 3 |
| 4 | 9 9 6 | 9 9 9 9 | 9 9 9 9 | 9 7 0 | 9 9 9 | 0 9 9 | 0 - - | 9 9 0 | 9 6 6 |
| 5 | 9 9 7 | 9 0 0 9 | 9 9 9 9 | 9 6 0 | 9 9 9 | 0 9 9 | 9 - - | 9 9 0 | 9 9 8 |
| 6 | 9 9 5 | 9 9 9 9 | 9 9 9 9 | 9 8 0 | 0 0 | 9 9 | - - | 9 9 0 | 9 9 6 |
| 7 | 9 9 9 | 9 9 9 9 | 9 9 9 9 | 9 5 0 | 9 9 9 | 9 9 9 | 0 - - | 9 8 0 | 9 9 0 |
| 8 | 9 9 8 | 9 9 9 9 | 9 9 9 9 | 9 4 0 | 9 9 9 | 0 9 8 | 0 - 9 | 9 9 9 | 9 7 0 |
| 9 | 9 9 9 | 9 9 9 9 | 9 9 9 9 | 9 8 5 | 9 9 9 | 4 9 9 | 8 - - | 9 0 | 8 7 0 |
| 10 | 9 5 | 9 9 9 9 | 9 9 9 9 | 9 9 5 | 9 9 9 | 0 9 0 | 0 - 8 | 0 0 | 2 0 |
| 11 | 9 9 9 | 9 9 9 9 | 9 9 9 9 | 9 7 0 | 0 9 9 | 0 9 9 | 0 - - | 9 9 5 | 9 9 |
| 12 | | | | | | | | | |
| 13 | 2 | 9 9 x | 9 9 x | 9 2 | 5 0 | 0 0 | 5 0 | 4 0 | 3 0 |
| 14 | 9 9 0 | 9 9 9 9 | 9 9 9 9 | 0 | 0 | 0 | 0 | 0 | 9 0 |
| 15 | 9 9 0 | 9 9 7 9 | 9 9 8 9 | 9 7 | 0 | 0 | 0 | 0 | 5 |
| 16 | | | | | | | | | |
| 17 | | | | | | | | | |
| 18 | 9 9 9 | 9 9 9 9 | 9 9 9 * | 9 * * | 0 | 0 | 0 | 9 9 0 | 9 5 0 |
| 19 | 9 9 2 | 9 9 9 9 | 9 9 9 * | 9 * * | 9 * * | 0 * * | 9 * * | 9 9 4 | 9 4 0 |
| 20 | | | | | | | | | |

## Claims

### Claims for the following Contracting States : AT, BE, CH, DE, DK, FR, GB, GR, IT, LI, LU, NL, SE

1. An N-acylated arylpyrrole compound represented by the structural formula I:

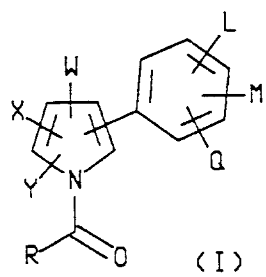

(I)

wherein

X is H, F, Cl, Br, I, or $CF_3$;
Y is F, Cl, Br, I, or $CF_3$;
W is CN, or $NO_2$;
L is H, F, Cl or Br;
M and Q are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$, $R_2CO$ or $NR_3R_4$;
and when M and Q are attached to adjacent carbon atoms in the phenyl ring and taken with the carbon atoms to which they are attached, they may form a ring in which MQ represents the structure:
$-OCH_2O-$, $-OCF_2O-$ or

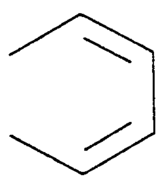

;

Z is S(O)n or O;
$R_1$ is H, F, $CHF_2$, CHFCl, or $CF_3$;
$R_2$ is $C_1$-$C_3$ alkyl, $C_1$-$C_3$ alkoxy, or $NR_3R_4$;
$R_3$ is H or $C_1$-$C_3$ alkyl;
$R_4$ is H, $C_1$-$C_3$ alkyl, or $R_5CO$;
$R_5$ is H or $C_1$-$C_3$ alkyl;
n is an integer of 0, 1 or 2; and
R is $C_1$-$C_6$ alkyl optionally substituted with
one to three halogen atoms,
one hydroxy,
one cyano,
one or two $C_1$-$C_4$ alkoxy groups optionally substituted with one to three halogen atoms,
one $C_1$-$C_4$ alkylthio,
one phenyl group optionally substituted with one to three halogen atoms, one to three $C_1$-$C_4$ alkyl groups or one to three $C_1$-$C_4$ alkoxy groups,
one phenoxy group optionally substituted with one to three halogen atoms, one to three $C_1$-$C_4$ alkyl groups or one to three $C_1$-$C_4$ alkoxy groups,
one benzyloxy group optionally substituted on the phenyl ring with one to three halogen atoms, one to three $C_1$-$C_4$ alkyl groups or one to three $C_1$-$C_4$ alkoxy groups,
one $C_1$-$C_6$ alkyl carbonyloxy group optionally substituted with one to three halogen atoms,

one $C_2$-$C_6$ alkenylcarbonyloxy group optionally substituted with one to three halogen atoms,

one phenylcarbonyloxy group optionally substituted with one to three halogen atoms, one to three $C_1$-$C_4$ alkyl groups or one to three $C_1$-$C_4$ alkoxy groups,

one $C_1$-$C_6$ alkoxycarbonyl group optionally substituted with one to three halogen atoms, or one to three $C_1$-$C_4$ alkoxy groups, or

one benzyloxycarbonyl group optionally substituted on the phenyl ring with one to three halogen atoms, one to three $C_1$-$C_4$ alkyl groups or one to three $C_1$-$C_4$ alkoxy groups,

$C_2$-$C_6$ alkenyl optionally substituted with one to three halogen atoms or one phenyl group,

$C_3$-$C_6$ alkynyl optionally substituted with one to three halogen atoms or one phenyl group,

$C_3$-$C_6$ cycloalkyl group,

phenyl optionally substituted with one to three halogen atoms, one to three $C_1$-$C_4$ alkyl groups, one $C_5$-$C_{12}$ alkyl group, one to two $C_1$-$C_4$ alkoxy groups, or one phenoxy, $C_1$-$C_4$ alkylthio, trialkylsilyl, $C_1$-$C_4$ alkylsulfinyl, $C_1$-$C_4$ alkylsulfonyl, carbo-$C_1$-$C_4$-alkoxy, carboxy, $CF_3$, CN, $NO_2$, di($C_1$-$C_4$ alkyl)amino, or $C_1$-$C_4$ alkanoylamino,

phenoxy optionally substituted with one to three halogen atoms, one or two $C_1$-$C_4$ alkyl groups, one or two $C_1$-$C_4$ alkoxy groups, trialkylsilyl, $CF_3$, CN, $NO_2$, or di($C_1$-$C_4$ alkyl)amino groups, or $C_1$-$C_4$ alkanoylamino,

1- or 2-naphthyl,

2-, 3-, or 4-pyridyl optionally substituted with one to three halogen atoms,

a heteroaromatic ring 5-membered ring containing an oxygen, nitrogen, or a sulfur atom, and optionally substituted with one to three halogen atoms,

$C_1$-$C_6$ alkoxy group optionally substituted with one to three halogen atoms; or

$C_2$-$C_6$ alkenyloxy group optionally substituted with one to three halogen atoms.

**2.** The compound according to claim 1 wherein the compound has the formula

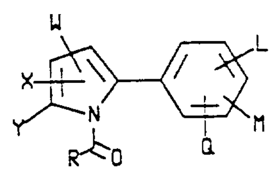

and wherein R is

phenyl optionally substituted with one to three halogen atoms, one to three $C_1$-$C_4$ alkyl groups, one to two $C_1$-$C_4$ alkoxy groups, or one phenoxy, $C_1$-$C_4$ alkylthio, trialkylsilyl, $C_1$-$C_4$ alkylsulfinyl, $C_1$-$C_4$ alkylsulfonyl, carbo-$C_1$-$C_4$-alkoxy, carboxy, $CF_3$, CN, $NO_2$, di($C_1$-$C_4$ alkyl)amino, or $C_1$-$C_4$ alkanoylamino,

1-naphthyl or 2-naphthyl,

2-, 3-, or 4-pyridyl optionally substituted with one to three halogen atoms, or

a heteroaromatic ring 5-membered ring containing an oxygen, nitrogen, or a sulfur atom, and optionally substituted with one to three halogen atoms.

**3.** The compound according to claim 1, wherein said N-acylated arylpyrrole is:

1-benzoyl-4-bromo-2-(p-chlorophenyl)-5-(trifluoromethyl)pyrrole-3-carbonitrile;
4-bromo-2-(p-chlorophenyl)-1-methacryloyl-5-(trifluoromethyl)pyrrole-3-carbonitrile;
4-bromo-2-(p-chlorophenyl)-1-o-toluoyl-5-(trifluoromethyl)pyrrole-3-carbonitrile;
4-bromo-1-(m-chlorobenzoyl)-1-(p-chlorophenyl)-5-(trifluoromethyl)pyrrole-3-carbonitrile;
4-bromo-2-(p-chlorophenyl)-1-(2-furoyl)-5-(trifluoromethyl)pyrrole-3-carbonitrile;
4-bromo-2-(p-chlorophenyl)-1-p-toluoyl-5-(trifluoromethyl)pyrrole-3-carbonitrile;
4-bromo-2-(p-chlorophenyl)-5-(trifluoromethyl)-1-(α,α,α-trifluoro-p-toluoyl)pyrrole-3-carbonitrile;
4-bromo-2-(p-chlorophenyl)-1-(p-nitrobenzoyl)-5-(trifluoromethyl)pyrrole-3-carbonitrile;
phenyl 3-bromo-5-(p-chlorophenyl)-4-cyano-2-(trifluoromethyl)pyrrole-1-carboxylate;

4-bromo-1-(p-chlorobenzoyl)-2-(p-chlorophenyl)-5-(trifluoromethyl)pyrrole-3-carbonitrile;
4-bromo-2-(p-chlorophenyl)-1-(cyclohexylcarbonyl)-5-(trifluoromethyl)pyrrole-3-carbonitrile;
1-benzoyl-4-bromo-2-chloro-5-(p-chlorophenyl)pyrrole-3-carbonitrile;
4-bromo-2-(p-chlorophenyl)-1-pivaloyl-5-(trifluoromethyl)pyrrole-3-carbonitrile;
1-acetyl-4,5-dichloro-2-(3,4-dichlorophenyl)pyrrole-3-carbonitrile;
phenyl 2,3-dibromo-4-cyano-5-(α,α,α-trifluoro-p-tolyl)pyrrole-1-carboxylate;
4-bromo-2-(p-chlorophenyl)-1-(1-naphthoyl)-5-(trifluoromethyl)pyrrole-3-carbonitrile;
4-bromo-2-(p-chlorophenyl)-1-(m-fluorobenzoyl)-5-(trifluoromethyl)pyrrole-3-carbonitrile;
4-bromo-2-(p-chlorophenyl)-1-(3,4-dichlorobenzoyl)-5-(trifluoromethyl)pyrrole-3-carbonitrile;
4-bromo-1-(p-tert-butylbenzoyl)-2-(p-chlorophenyl)-5-(trifluoromethyl)pyrrole-3-carbonitrile; or
1-benzoyl-4-bromo-2-(p-bromophenyl)-5-(trifluoromethyl)pyrrole-3-carbonitrile.

**4.** The compound according to claim 3, wherein the N-acylated arylpyrrole is 1-benzoyl-4-bromo-2-(p-chlorophenyl)-5-(trifluoromethyl)pyrrole-3-carbonitrile; 4-bromo-2-(p-chlorophenyl)-1-methacryloyl)-5-(trifluoromethyl)pyrrole-3-carbonitrile; or 4-bromo-2-(p-chlorophenyl)-1-o-toluoyl-5-(trifluoromethyl)-pyrrole-3-carbonitrile.

**5.** A compound according to claim 1 for use in a method for controlling insects, acarina, nematodes, fungi or mollusks which comprises contacting said insects, acarina, nematodes, fungi or mollusks, their breeding grounds, food supply or habitat with an insecticidally, acaricidally, nematicidally, fungicidally or molluscicidally effective amount of an N-acylated arylpyrrole.

**6.** The method according to claim 5 wherein said formula I N-acylated arylpyrrole is applied to said insect, acarid, nematode, fungi or mollusk pests, their breeding grounds, food supply or habitat in sufficient amount to provide a rate of from 0.1 kg/ha to about 4.0 kg/ha of active ingredient.

**7.** The method according to claim 5, wherein said N-acylated arylpyrrole has the formula

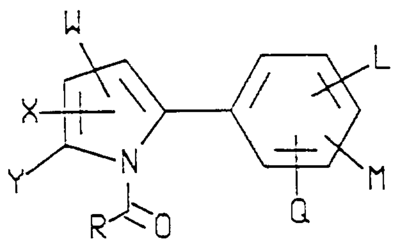

and wherein R is as defined in claim 2.

**8.** A process for the preparation of N-acylated arylpyrroles as defined in claim 1, which comprises reacting an arylpyrrole having the structure:

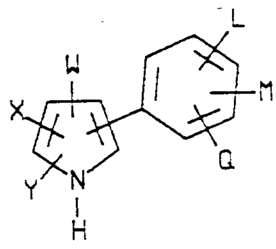

wherein L, M, Q, W, X and Y are as described in claim 1, with an excess of an alkali metal hydride or alkali metal t-butoxide, in the presence of an anhydrous inert organic solvent to form a first mixture, adding the appropriately substituted carbonyl chloride having the structure: RCOCl, wherein R is as described in claim 1, to form a second mixture, heating said second mixture to form said N-acylated arylpyrrole.

**9.** The process according to claim 8, which further comprises heating said first mixture to refluxing temperature, then cooling said first mixture to a temperature between about 20 °C and 30 °C and wherein said second mixture is heated to refluxing temperature.

**10.** The process according to claim 8, wherein the alkali metal hydride is sodium hydride, the organic solvent is tetrahydrofuran, dimethylformamide, or dimethylsulfoxide and the reaction of the arylpyrrole with the sodium hydride is conducted under a blanket of nitrogen.

**Claims for the following Contracting State : ES**

**1.** A process for preparing an N-acylated arylpyrrole compound represented by the structural formula I:

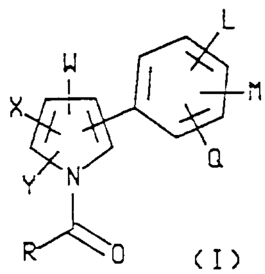

(I)

wherein

| | | |
|---|---|---|
| X | is H, F, Cl, Br, I, or $CF_3$; |
| Y | is F, Cl, Br, I, or $CF_3$; |
| W | is CN, or $NO_2$; |
| L | is H, F, Cl or Br; |
| M and Q | are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$, $R_2CO$ or $NR_3R_4$; and when M and Q are attached to adjacent carbon atoms in the phenyl ring and taken with the carbon atoms to which they are attached, they may form a ring in which MQ represents the structure: $-OCH_2O-$, $-OCF_2O-$ or 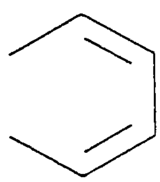 ; |

| | |
|---|---|
| Z | is S(O)n or O; |
| $R_1$ | is H, F, $CHF_2$, CHFCl, or $CF_3$; |
| $R_2$ | is $C_1$-$C_3$ alkyl, $C_1$-$C_3$ alkoxy, or $NR_3R_4$; |
| $R_3$ | is H or $C_1$-$C_3$ alkyl; |
| $R_4$ | is H, $C_1$-$C_3$ alkyl, or $R_5CO$; |
| $R_5$ | is H or $C_1$-$C_3$ alkyl; |
| n | is an integer of 0, 1 or 2; and |
| R | is $C_1$-$C_6$ alkyl optionally substituted with one to three halogen atoms, one hydroxy, one cyano, one or two $C_1$-$C_4$ alkoxy groups optionally substituted with one to three halogen atoms, one $C_1$-$C_4$ alkylthio, one phenyl group optionally substituted with one to three halogen atoms, one to three $C_1$-$C_4$ alkyl groups or one to three $C_1$-$C_4$ alkoxy groups, |

one phenoxy group optionally substituted with one to three halogen atoms, one to three $C_1$-$C_4$ alkyl groups or one to three $C_1$-$C_4$ alkoxy groups,

one benzyloxy group optionally substituted on the phenyl ring with one to three halogen atoms, one to three $C_1$-$C_4$ alkyl groups or one to three $C_1$-$C_4$ alkoxy groups,

one $C_1$-$C_6$ alkyl carbonyloxy group optionally substituted with one to three halogen atoms,

one $C_2$-$C_6$ alkenylcarbonyloxy group optionally substituted with one to three halogen atoms,

one phenylcarbonyloxy group optionally substituted with one to three halogen atoms, one to three $C_1$-$C_4$ alkyl groups or one to three $C_1$-$C_4$ alkoxy groups,

one $C_1$-$C_6$ alkoxycarbonyl group optionally substituted with one to three halogen atoms, or one to three $C_1$-$C_4$ alkoxy groups, or

one benzyloxycarbonyl group optionally substituted on the phenyl ring with one to three halogen atoms, one to three $C_1$-$C_4$ alkyl groups or one to three $C_1$-$C_4$ alkoxy groups,

$C_2$-$C_6$ alkenyl optionally substituted with one to three halogen atoms or one phenyl group,

$C_3$-$C_6$ alkynyl optionally substituted with one to three halogen atoms or one phenyl group,

$C_3$-$C_6$ cycloalkyl group,

phenyl optionally substituted with one to three halogen atoms, one to three $C_1$-$C_4$ alkyl groups, one $C_5$-$C_{12}$ alkyl group, one to two $C_1$-$C_4$ alkoxy groups, or one phenoxy, $C_1$-$C_4$ alkylthio, trialkylsilyl, $C_1$-$C_4$ alkylsulfinyl, $C_1$-$C_4$ alkylsulfonyl, carbo-$C_1$-$C_4$-alkoxy, carboxy, $CF_3$, CN, $NO_2$, di($C_1$-$C_4$ alkyl)amino, or $C_1$-$C_4$ alkanoylamino,

phenoxy optionally substituted with one to three halogen atoms, one or two $C_1$-$C_4$ alkyl groups, one or two $C_1$-$C_4$ alkoxy groups, trialkylsilyl, $CF_3$, CN, $NO_2$, or di($C_1$-$C_4$ alkyl)amino groups, or $C_1$-$C_4$ alkanoylamino,

1- or 2-naphthyl,

2-, 3-, or 4-pyridyl optionally substituted with one to three halogen atoms,

a heteroaromatic ring 5-membered ring containing an oxygen, nitrogen, or a sulfur atom, and optionally substituted with one to three halogen atoms,

$C_1$-$C_6$ alkoxy group optionally substituted with one to three halogen atoms; or

$C_2$-$C_6$ alkenyloxy group optionally substituted with one to three halogen atoms,

which comprises reacting an arylpyrrole having the structure:

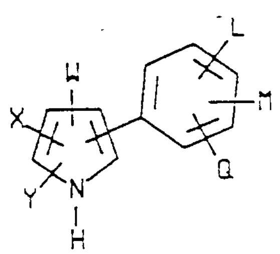

wherein L, M, Q, W, X and Y are as described above with an excess of an alkali metal hydride or alkali metal t-butoxide, in the presence of an anhydrous inert organic solvent to form a first mixture, adding the appropriately substituted carbonyl chloride having the structure: RCOCl, wherein R is as described above, to form a second mixture, heating said second mixture to form said N-acylated arylpyrrole.

2. The process according to claim 1, which further comprises heating said first mixture to refluxing temperature, then cooling said first mixture to a temperature between about 20 °C and 30 °C and wherein said second mixture is heated to refluxing temperature.

3. The process according to claim 1, wherein the alkali metal hydride is sodium hydride, the organic solvent is tetrahydrofuran, dimethylformamide, or dimethylsulfoxide and the reaction of the arylpyrrole with the sodium hydride is conducted under a blanket of nitrogen.

**4.** The process according to claim 1 wherein the compound has the formula

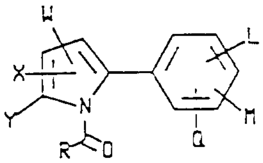

and wherein R is

phenyl optionally substituted with one to three halogen atoms, one to three $C_1$-$C_4$ alkyl groups, one to two $C_1$-$C_4$ alkoxy groups, or one phenoxy, $C_1$-$C_4$ alkylthio, trialkylsilyl, $C_1$-$C_4$ alkylsulfinyl, $C_1$-$C_4$ alkylsulfonyl, carbo-$C_1$-$C_4$-alkoxy, carboxy, $CF_3$, CN, $NO_2$, di($C_1$-$C_4$ alkyl)amino, or $C_1$-$C_4$ alkanoylamino,

1-naphthyl or 2-naphthyl,

2-, 3-, or 4-pyridyl optionally substituted with one to three halogen atoms, or

a heteroaromatic ring 5-membered ring containing an oxygen, nitrogen, or a sulfur atom, and optionally substituted with one to three halogen atoms.

**5.** The process according to claim 1, wherein said N-acylated arylpyrrole is:

1-benzoyl-4-bromo-2-(p-chlorophenyl)-5-(trifluoromethyl)pyrrole-3-carbonitrile;
4-bromo-2-(p-chlorophenyl)-1-methacryloyl-5-(trifluoromethyl)pyrrole-3-carbonitrile;
4-bromo-2-(p-chlorophenyl)-1-o-toluoyl-5-(trifluoromethyl)pyrrole-3-carbonitrile;
4-bromo-1-(m-chlorobenzoyl)-1-(p-chlorophenyl)-5-(trifluoromethyl)pyrrole-3-carbonitrile;
4-bromo-2-(p-chlorophenyl)-1-(2-furoyl)-5-(trifluoromethyl)pyrrole-3-carbonitrile;
4-bromo-2-(p-chlorophenyl)-1-p-toluoyl-5-(trifluoromethyl)pyrrole-3-carbonitrile;
4-bromo-2-(p-chlorophenyl)-5-(trifluoromethyl)-1-($\alpha$,$\alpha$,$\alpha$-trifluoro-p-toluoyl)pyrrole-3-carbonitrile;
4-bromo-2-(p-chlorophenyl)-1-(p-nitrobenzoyl)-5-(trifluoromethyl)pyrrole-3-carbonitrile;
phenyl 3-bromo-5-(p-chlorophenyl)-4-cyano-2-(trifluoromethyl)pyrrole-1-carboxylate;
4-bromo-1-(p-chlorobenzoyl)-2-(p-chlorophenyl)-5-(trifluoromethyl)pyrrole-3-carbonitrile;
4-bromo-2-(p-chlorophenyl)-1-(cyclohexylcarbonyl)-5-(trifluoromethyl)pyrrole-3-carbonitrile;
1-benzoyl-4-bromo-2-chloro-5-(p-chlorophenyl)pyrrole-3-carbonitrile;
4-bromo-2-(p-chlorophenyl)-1-pivaloyl-5-(trifluoromethyl)pyrrole-3-carbonitrile;
1-acetyl-4,5-dichloro-2-(3,4-dichlorophenyl)pyrrole-3-carbonitrile;
phenyl 2,3-dibromo-4-cyano-5-($\alpha$,$\alpha$,$\alpha$-trifluoro-p-tolyl)pyrrole-1-carboxylate;
4-bromo-2-(p-chlorophenyl)-1-(1-naphthoyl)-5-(trifluoromethyl)pyrrole-3-carbonitrile;
4-bromo-2-(p-chlorophenyl)-1-(m-fluorobenzoyl)-5-(tri fluoromethyl)pyrrole-3-carbonitrile;
4-bromo-2-(p-chlorophenyl)-1-(3,4-dichlorobenzoyl)-5-(trifluoromethyl)pyrrole-3-carbonitrile;
4-bromo-1-(p-tert-butylbenzoyl)-2-(p-chlorophenyl)-5-(trifluoromethyl)pyrrole-3-carbonitrile; or
1-benzoyl-4-bromo-2-(p-bromophenyl)-5-(trifluoromethyl)pyrrole-3-carbonitrile.

**6.** The process according to claim 5, wherein the N-acylated arylpyrrole is 1-benzoyl-4-bromo-2-(p-chlorophenyl)-5-(trifluoromethyl)pyrrole-3-carbonitrile; 4-bromo-2-(p-chlorophenyl)-1-methacryloyl)-5-(trifluoromethyl)pyrrole-3-carbonitrile; or 4-bromo-2-(p-chlorophenyl)-1-o-toluoyl-5-(trifluoromethyl)-pyrrole-3-carbonitrile.

**7.** Use of a compound prepared according to claim 1 in a method for controlling insects, acarina, nematodes, fungi or mollusks which comprises contacting said insects, acarina, nematodes, fungi or mollusks, their breeding grounds, food supply or habitat with an insecticidally, acaricidally, nematicidally, fungicidally or molluscicidally effective amount of an N-acylated arylpyrrole.

**8.** The use according to claim 7 wherein said formula I N-acylated arylpyrrole is applied to said insect, acarid, nematode, fungi or mollusk pests, their breeding grounds, food supply or habitat in sufficient amount to provide a rate of from 0.1 kg/ha to about 4.0 kg/ha of active ingredient.

**9.** The use according to claim 7, wherein said N-acylated arylpyrrole has the formula

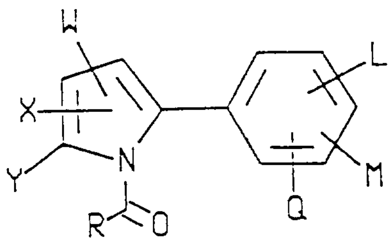

and wherein R is as defined in claim 4.

## Patentansprüche

**Patentansprüche für folgende Vertragsstaaten : AT, BE, CH, DE, DK, FR, GB, GR, IT, LI, LU, NL, SE**

**1.** N-acylierte Arylpyrrolverbindung der allgemeinen Strukturformel I:

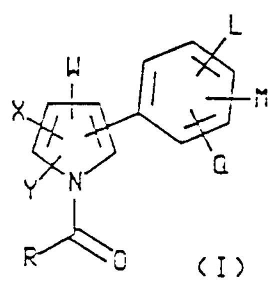

(I)

worin

| | |
|---|---|
| X | H, F, Cl, Br, I oder $CF_3$ ist; |
| Y | F, Cl, Br, I oder $CF_3$ ist; |
| W | CN oder $NO_2$ ist; |
| L | H, F, Cl oder Br ist; und |
| M und Q | jeweils unabhängig H, $C_1$-$C_3$-Alklyl, $C_1$-$C_3$-Alkoxy, $C_1$-$C_3$-Alkylthio, $C_1$-$C_3$-Alkylsulfinyl, $C_1$-$C_3$-Alkylsulfonyl, Cyano, F, Cl, Br, I, Nitro, $CF_3$, $R_1CF_2Z$, $R_2CO$ oder $NR_3R_4$ ist; und wenn M und Q an benachbarte Kohlenstoffatome des Phenylrings gebunden sind, können sie zusammen mit den Kohlenstoffatomen, an welche sie gebunden sind, einen Ring bilden, in welchem MQ folgende Struktur darstellt: |

$-OCH_2O-$, $-OCF_2O-$ oder

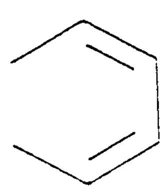

;

| | |
|---|---|
| Z | S(O)n oder O ist; |
| $R_1$ | H, F, $CHF_2$, CHFCl oder $CF_3$ ist; |
| $R_2$ | $C_1$-$C_3$-Alkyl, $C_1$-$C_3$-Alkoxy oder $NR_3R_4$ ist; |
| $R_3$ | H oder $C_1$-$C_3$-Alkyl ist; |
| $R_4$ | H, $C_1$-$C_3$-Alkyl oder $R_5CO$ ist; |
| $R_5$ | H oder $C_1$-$C_3$-Alkyl ist; und |
| n | eine ganze Zahl 0, 1 oder 2 ist; und |

R $C_1$-$C_6$-Alkyl, gegebenenfalls substituiert mit
einem bis drei Halogenatomen,
einer Hydroxygruppe,
einer Cyanogruppe,

einer oder zwei $C_1$-$C_4$-Alkoxygruppen, gegebenenfalls substituiert mit einem bis drei Halogenatomen,

einer $C_1$-$C_4$-Alkylthiogruppe,

einer Phenylgruppe, gegebenenfalls substituiert mit einem bis drei Halogenatomen, einer bis drei $C_1$-$C_4$-Alkylgruppen oder einer bis drei $C_1$-$C_4$-Alkoxygruppen,

einer Phenoxygruppe, gegebenenfalls substituiert mit einem bis drei Halogenatomen,
einer bis drei $C_1$-$C_4$-Alkylgruppen oder einer bis drei $C_1$-$C_4$-Alkoxygruppen,

einer Benzyloxygruppe, gegebenenfalls substituiert am Phenylring mit einem bis drei Halogenatomen,
einer bis drei $C_1$-$C_4$-Alkylgruppen oder einer bis drei $C_1$-$C_4$-Alkoxygruppen,

einer $C_1$-$C_6$-Alkylcarbonyloxygrupoe, gegebenenfalls substituiert mit einem bis drei Halogenatomen,

einer $C_2$-$C_6$-Alkenylcarbonyloxygruppe, gegebenenfalls substituiert mit einem bis drei Halogenatomen,

einer Phenylcarbonyloxygruppe, gegebenenfalls substituiert mit einem bis drei Halogenatomen, einer bis drei $C_1$-$C_4$-Alkylgruppen oder einer bis drei $C_1$-$C_4$-Alkoxygruppen,

einer $C_1$-$C_6$-Alkoxycarbonylgruppe, gegebenenfalls substituiert mit einem bis drei Halogenatomen oder einer bis drei $C_1$-$C_4$-Alkoxygruppen, oder

eine Benzyloxycarbonylgruppe, gegebenenfalls substituiert am Phenylring mit einem bis drei Halogenatomen, einer bis drei $C_1$-$C_4$-Alkylgruppen oder einer bis drei $C_1$-$C_4$-Alkoxygruppen,

$C_2$-$C_6$-Alkenyl, gegebenenfalls substituiert mit einem bis drei Halogenatomen oder einer Phenylgruppe,

$C_3$-$C_6$-Alkynyl, gegebenenfalls substituiert mit einem bis drei Halogenatomen oder einer Phenylgruppe, $C_3$-$C_6$-Cycloalkyl,

Phenyl, gegebenenfalls substituiert mit einem bis drei Halogenatomen, einer bis drei $C_1$-$C_4$-Alkylgruppen, einer $C_5$-$C_{12}$-Alkylgruppe, einer bis zwei $C_1$-$C_4$-Alkoxygruppen oder einer Phenoxy, $C_1$-$C_4$-Alkylthio, Trialkylsilyl, $C_1$-$C_4$-Alkylsulfinyl, $C_1$-$C_4$-Alkylsulfonyl, Carbo-$C_1$-$C_4$-alkoxy, Carboxy, $CF_3$, CN, $NO_2$, Di($C_1$-$C_4$-alkyl)amino oder $C_1$-$C_4$-Alkanoylamino,

Phenoxy, gegebenenfalls substituiert mit einem bis drei Halogenatomen, einer oder zwei $C_1$-$C_4$-Alkylgruppen, einer oder zwei $C_1$-$C_4$-Alkoxygruppen, Trialkylsilyl, $CF_3$, CN, $NO_2$ oder Di($C_1$-$C_4$-alkyl)aminogruppen oder $C_1$-$C_4$-Alkanoylamino,

1-Naphthyl oder 2-Naphthyl,

2-, 3- oder 4-Pyridyl, gegebenenfalls substituiert mit einem bis drei Halogenatomen,

ein heteroaromatischer 5-gliedriger Ring, enthaltend ein Sauerstoff-, Stickstoff- oder ein Schwefelatom und gegebenenfalls substituiert mit einem bis drei Halogenatomen,

$C_1$-$C_6$-Alkoxygruppe, gegebenenfalls substituiert mit einem bis drei Halogenatomen oder

$C_2$-$C_6$-Alkenyloxygruppe, gegebenenfalls substituiert mit einem bis drei Halogenatomen, ist.

2. Verbindung gemäß Anspruch 1, wobei die Verbindung die allgemeine Formel

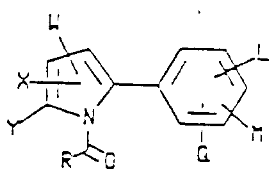

hat und worin R

Phenyl, gegebenenfalls substituiert mit einem bis drei Halogenatomen, einer bis drei $C_1$-$C_4$-Alkylgruppen, einer bis zwei $C_1$-$C_4$-Alkoxygruppen oder einer Phenoxy, $C_1$-$C_4$-Alkylthio, Trialkylsilyl, $C_1$-$C_4$-Alkylsulfinyl, $C_1$-$C_4$-Alkylsulfonyl, Carbo-$C_1$-$C_4$-alkoxy, Carboxy, $CF_3$, CN, $NO_2$, Di($C_1$-$C_4$-alkyl)-amino oder $C_1$-$C_4$-Alkanoylamino,

1-Naphthyl oder 2-Naphthyl,

2-, 3- oder 4-Pyridyl, gegebenenfalls substituiert mit einem bis drei Halogenatomen, oder

ein heteroaromatischer 5-gliedriger Ring, enthaltend ein Sauerstoff-, Stickstoff- oder ein Schwefelatom und gegebenenfalls substituiert mit einem bis drei Halogenatomen, ist.

3. Verbindung gemäß Anspruch 1, worin besagtes N-acyliertes Arylpyrrol
1-Benzoyl-4-bromo-2-(p-chlorophenyl)-5-(trifluoromethyl)pyrrol-3-carbonitril;
4-Bromo-2-(p-chlorophenyl)-1-methacryloyl-5-(trifluoromethyl)pyrrol-3-carbonitril;
4-Bromo-2-(p-chlorophenyl)-1-o-toluoyl-5-(trifluoromethyl)pyrrol-3-carbonitril;
4-Bromo-1-(m-chlorophenyl)-1-(p-chlorophenyl)-5-(trifluoromethyl)pyrrol-3-carbonitril;
4-Bromo-2-(p-chlorophenyl)-1-(2-furoyl)-5-(trifluoromethyl)pyrrol-3-carbonitril;
4-Bromo-2-(p-chlorophenyl)-1-p-toluoyl-5-(trifluoromethyl)pyrrol-3-carbonitril;
4-Bromo-2-(p-chlorophenyl)-5-(trifluoromethyl)-1-(a,a,a-trifluoro-p-toluoyl)pyrrol-3-carbo-nitril;
4-Bromo-2-(p-chlorophenyl)-1-(p-nitrobenzoyl)-5-(trifluoromethyl)pyrrol-3-carbonitril;
Phenyl-3-bromo-5-(p-chlorophenyl)-4-cyano-2-(trifluoromethyl)pyrrol-1-carboxylat;
4-Bromo-1-(p-chlorophenyl)-2-(p-chlorophenyl)-5-(trifluoromethyl)pyrrol-3-carbonitril;
4-Bromo-2-(p-chlorophenyl)-1-(cycloheylcar-bonyl)-5-(trifluoromethyl)pyrrol-3-carbonitril;
4-Bromo-2-(p-chlorophenyl)-1-pivaloyl-5-(trifluoromethyl)pyrrol-3-carbonitril;
1-Acetyl-4,5-dichloro-2-(3,4-dichlorophenyl)-pyrrol-3-carbonitril;
Phenyl-2,3-dibromo-4-cyano-5-(a,a,a-trifluoro-p-tolyl)pyrrol-3-carboxylat;
4-Bromo-2-(p-chlorophenyl)-1-(1-naphthoyl)-5-(trifluoromethyl)pyrrol-3-carbonitril;
4-Bromo-2-(p-chlorophenyl)-1-(m-fluorobenzoyl)-5-(trifluoromethyl)pyrrol-3-carbonitril;
4-Bromo-2-(p-chlorophenyl)-1-(3,4-dichlorobenzoyl)-5-(trifluoronethyl)pyrrol-3-carbonitril;
1-Benzoyl-4,5-dichloro-2-(3,4-dichlorophenyl)-pyrrol-3-carbonitril;
4-Bromo-1-(p-tert-butylbenzoyl)-2-(p-chloro-phenyl)-5-(trifluoromethyl)pyrrol-3-carbo-nitril; oder
1-Benzoyl-4-bromo-2-(p-chlorophenyl)-5-(trifluoromethyl)pyrrol-3-carbonitril; ist.

4. Verbindung gemäß Anspruch 3, worin das N-acylierte Arylpyrrol
1-Benzoyl-4-bromo-2-(p-chlorophenyl)-5-(trifluoromethyl)pyrrol-3-carbonitril;
4-Bromo-2-(p-chlorophenyl)-1-methacryloyl-5-(trifluoromethyl)pyrrol-3-carbonitril; oder
4-Bromo-2-(p-chlorophenyl)-1-o-toluoyl-5-(trifluoromethyl)pyrrol-3-carbonitril; ist.

5. Verbindung gemäß Anspruch 1 zur Verwendung in einem Verfahren zur Bekämpfung von Insekten, Akariden, Nematoden, Pilzen oder Mollusken, umfassend das Inberührungbringen der besagten Insekten, Akariden, Nematoden, Pilze oder Mollusken, ihre Fortpflanzungsstätten, Nahrung oder Lebensraum mit einer insektizid, akarizid, nematizid, fungizid oder molluskizid wirksamen Menge eines N-acylierten Arylpyrrol.

6. Verfahren gemäß Anspruch 5, worin besagtes N-acyliertes Arylpyrrol auf besagte Insekten, Akariden, Nematoden, Pilze oder Mollusken, ihre Fortpflanzungsstätten, Nahrung oder Lebensraum in ausreichender Menge angewendet wird, um eine aktive Bestandteilmenge von 0.1 kg/ha bis 4.0 kg/ha bereitzustellen.

7. Verfahren gemäß Anspruch 5, worin besagtes N-acyliertes Arylpyrrol die allgemeine Formel

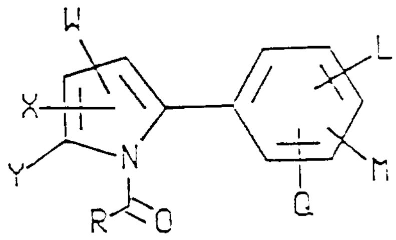

hat und worin R wie in Anspruch 2 definiert ist.

8. Verfahren zur Herstellung wie in Anspruch 1 definierter N-acylierter Arylpyrrole, umfassend die Reaktion eines Arylpyrrols mit der allgemeinen Struktur:

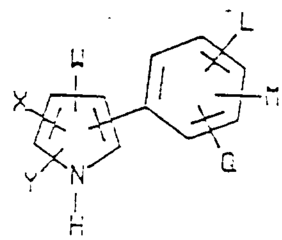

worin L, M, Q, W, X, und Y wie in Anspruch 1 beschrieben sind, mit einem Überschuß eines Alkalimetallhydrids oder Alkalimetal-t-butoxids in Gegenwart eines wasserfreien, inerten, organischen Lösungsmittels um ein erstes Gemisch zu bilden;
Hinzufügen des in geeigneter Weise substituierten Carbonylchlorids mit der allgemeinen Struktur: RCOCl, worin R wie in Anspruch 1 beschrieben ist, um ein zweites Gemisch zu bilden; Erhitzen des besagten zweiten Gemischs, um besagtes N-acyliertes Arylpyrrol zu bilden.

9. Verfahren gemäß Anspruch 8, das weiterhin umfaßt Erhitzen besagtes erstes Gemisch auf Rückflußtemperatur, dann Kühlen des besagten ersten Gemischs auf eine Temperatur zwischen ungefähr 20 °C bis 30 °C und worin besagtes zweites Gemisch auf Rückflußtemperatur erhitzt wird.

10. Verfahren gemäß Anspruch 8, worin das Alkalimetallhydrid Natriumhydrid ist, das organische Lösungsmittel Tetrahydrofuran, Dimethylformamid oder Dimethylsulfoxid ist und die Reaktion des Arylpyrrols mit dem Natriumhydrid unter einer Stickstoffdecke durchgeführt wird.

**Patentansprüche für folgenden Vertragsstaat : ES**

1. Verfahren zur Herstellung einer N-acylierten Arylpyrrolverbindung der allgemeinen Strukturformel I:

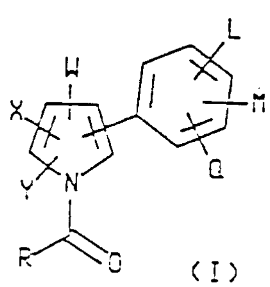

(I)

worin

| | |
|---|---|
| X | H, F, Cl, Br, I oder $CF_3$ ist; |
| Y | F, Cl, Br, I oder $CF_3$ ist; |
| W | CN oder $NO_2$ ist; |
| L | H, F, Cl oder Br ist; und |
| M und Q | jeweils unabhängig H, $C_1$-$C_3$-Alklyl, $C_1$-$C_3$-Alkoxy, $C_1$-$C_3$-Alkylthio, $C_1$-$C_3$-Alkylsulfinyl, $C_1$-$C_3$-Alkylsulfonyl, Cyano, F, Cl, Br, I, Nitro, $CF_3$, $R_1CF_2Z$, $R_2CO$ oder $NR_3R_4$ ist; und wenn M und Q an benachbarte Kohlenstoffatome des Phenylrings gebunden sind, können sie zusammen mit den Kohlenstoffatomen, an welche sie gebunden sind, einen Ring bilden, in welchem MQ folgende Struktur darstellt: -$OCH_2O$-, -$OCF_2O$- oder 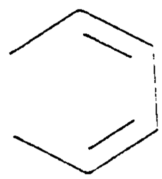 ; |
| Z | S(O)n oder O ist; |
| $R_1$ | H, F, $CHF_2$, CHFCl oder $CF_3$ ist; |
| $R_2$ | $C_1$-$C_3$-Alkyl, $C_1$-$C_3$-Alkoxy oder $NR_3R_4$ ist; |
| $R_3$ | H oder $C_1$-$C_3$-Alkyl ist; |
| $R_4$ | H, $C_1$-$C_3$-Alkyl oder $R_5CO$ ist; |
| $R_5$ | H oder $C_1$-$C_3$-Alkyl ist; und |
| n | eine ganze Zahl 0, 1 oder 2 ist; und |
| R | $C_1$-$C_6$-Alkyl ist, wahlweise substituiert mit |

einem bis drei Halogenatomen,
einer Hydroxygruppe,
einer Cyanogruppe,

einer oder zwei $C_1$-$C_4$-Alkoxygruppen, wahlweise substituiert mit einem bis drei Halogenatomen,

einer $C_1$-$C_4$-Alkylthiogruppe,

einer Phenylgruppe, wahlweise substituiert mit einem bis drei Halogenatomen, einer bis drei $C_1$-$C_4$-Alkylgruppen oder einer bis drei $C_1$-$C_4$-Alkoxygruppen,

einer Phenoxygruppe, wahlweise substituiert mit einem bis drei Halogenatomen, einer bis drei $C_1$-$C_4$-Alkylgruppen oder einer bis drei $C_1$-$C_4$-Alkoxygruppen,

einer Benzyloxygruppe, wahlweise substituiert am Phenylring mit einem bis drei Halogenatomen, einer bis drei $C_1$-$C_4$-Alkylgruppen oder einer bis drei $C_1$-$C_4$-Alkoxygruppen,

einer $C_1$-$C_6$-Alkylcarbonyloxygruppe, wahlweise substituiert mit einem bis drei Halogenatomen,

einer $C_2$-$C_6$-Alkenylcarbonyloxygruppe, wahlweise substituiert mit einem bis drei Halogenatomen,

einer Phenylcarbonyloxygruppe, wahlweise substituiert mit einem bis drei Halogenatomen, einer bis drei $C_1$-$C_4$-Alkylgruppen oder einer bis drei $C_1$-$C_4$-Alkoxygruppen,

einer $C_1$-$C_6$-Alkoxycarbonylgruppe, wahlweise substituiert mit einem bis drei Halogenatomen oder einer bis drei $C_1$-$C_4$-Alkoxygruppen, oder

eine Benzyloxycarbonylgruppe, wahlweise substituiert am Phenylring mit einem bis drei Halogenatomen, einer bis drei $C_1$-$C_4$-Alkylgruppen oder einer bis drei $C_1$-$C_4$-Alkoxygruppen,

$C_2$-$C_6$-Alkenyl, wahlweise substituiert mit einem bis drei Halogenatomen oder einer Phenylgruppe,

$C_3$-$C_6$-Alkynyl, wahlweise substituiert mit einem bis drei Halogenatomen oder einer Phenylgruppe,

$C_3$-$C_6$-Cycloalkyl,

Phenyl, wahlweise substituiert mit einem bis drei Halogenatomen, einer bis drei $C_1$-$C_4$-Alkylgruppen, einer $C_5$-$C_{12}$-Alkylgruppe, einer bis zwei $C_1$-$C_4$-Alkoxygruppen oder einer Phenoxy, $C_1$-$C_4$-Alkylthio, Trialkylsilyl, $C_1$-$C_4$-Alkylsulfinyl, $C_1$-$C_4$-Alkylsulfonyl, Carbo-$C_1$-$C_4$-alkoxy, Carboxy, $CF_3$, CN, $NO_2$, Di($C_1$-$C_4$-alkyl)amino oder $C_1$-$C_4$-Alkanoylamino,

Phenoxy, wahlweise substituiert mit einem bis drei Halogenatomen, einer oder zwei $C_1$-$C_4$-Alkylgruppen, einer oder zwei $C_1$-$C_4$-Alkoxygruppen, Trialkylsilyl, $CF_3$, CN, $NO_2$ oder Di($C_1$-$C_4$-alkyl)aminogruppen oder $C_1$-$C_4$-Alkanoylamino,

1-Naphthyl oder 2-Naphthyl,

2-, 3- oder 4-Pyridyl, wahlweise substituiert mit einem bis drei Halogenatomen,

ein heteroaromatischer 5-gliedriger Ring, enthaltend ein Sauerstoff-, Stickstoff- oder ein Schwefelatom und wahlweise substituiert mit einem bis drei Halogenatomen,

$C_1$-$C_6$-Alkoxygruppe, wahlweise substituiert mit einem bis drei Halogenatomen oder

$C_2$-$C_6$-Alkenyloxygruppe, wahlweise substituiert mit einem bis drei Halogenatomen,

$C_2$-$C_6$-Alkenyloxygruppe, gegebenenfalls substituiert mit einem bis drei Halogenatomen,

umfassend die Reaktion eines Arylpyrrols mit der allgemeinen Struktur:

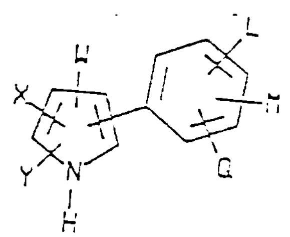

worin L, M, Q, W, X und Y wie oben beschrieben sind, mit einem Überschuß eines Alkalimetallhydrids oder Alkalimetall-t-butoxids in Gegenwart eines eines inerten eines wasserfreien, inerten, organischen Lösungsmittels, um ein erstes Gemisch zu bilden, das Zugeben des in geeigneter Weise substituierten Carbonylchlorids mit der allgemeinen Struktur: ROCl, worin R wie oben beschrieben ist, um ein zweites Gemisch zu bilden und das Erhitzen des besagten zweiten Gemischs, um das besagte N-acylierte Arylpyrrol zu bilden.

**2.** Verfahren gemäß Anspruch 1, das weiterhin umfaßt das Erhitzen des ersten Gemischs auf Rückflußtemperatur, dann das Abkühlen des besagten Gemischs auf eine Temperatur zwischen ungefähr 20° C und 30° C und worin das besagte zweite Gemisch auf Rückflußtemperatur erhitzt wird.

**3.** Verfahren gemäß Anspruch 1, worin das Alkalimetallhydrid Natriumhydrid ist, das organische Lösungsmittel Tetrahydrofuran, Dimethylformamid oder Dimethylsulfoxid ist und die Reaktion des Arylpyrrols mit dem Natriumhydrid unter einer Stickstoffdecke durchgeführt wird.

**4.** Verfahren gemäß Anspruch 1, worin die Verbindung die allgemeine Formel

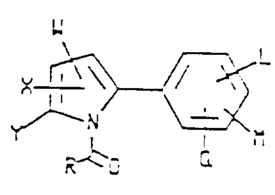

hat und worin R

Phenyl, gegebenenfalls substituiert mit einem bis drei Halogenatomen, einer bis drei $C_1$-$C_4$-Alkylgruppen, einer bis zwei $C_1$-$C_4$-Alkoxygruppen oder einer Phenoxy, $C_1$-$C_4$-Alkylthio, Trialkylsilyl, $C_1$-$C_4$-Alkylsulfinyl, $C_1$-$C_4$-Alkylsulfonyl, Carbo-$C_1$-$C_4$-alkoxy, Carboxy, $CF_3$, CN, $NO_2$, Di($C_1$-$C_4$-alkyl)-

amino oder $C_1$-$C_4$-Alkanoylamino,
1-Naphthyl oder 2-Naphthyl,
2-, 3- oder 4-Pyridyl, gegebenenfalls substituiert mit einem bis drei Halogenatomen, oder
ein heteroaromatischer 5-gliedriger Ring, enthaltend ein Sauerstoff-, Stickstoff- oder ein Schwefelatom und gegebenenfalls substituiert mit einem bis drei Halogenatomen, ist.

**5.** Verfahren gemäß Anspruch 1, worin besagtes N-acyliertes Arylpyrrol
1-Benzoyl-4-bromo-2-(p-chlorophenyl)-5-(trifluoromethyl)pyrrol-3-carbonitril;
4-Bromo-2-(p-chlorophenyl)-1-methacryloyl-5-(trifluoromethyl)pyrrol-3-carbonitril;
4-Bromo-2-(p-chlorophenyl)-1-o-toluoyl-5-(trifluoromethyl)pyrrol-3-carbonitril;
4-Bromo-1-(m-chlorophenyl)-1-(p-chlorophenyl)-5-(trifluoromethyl)pyrrol-3-carbonitril;
4-Bromo-2-(p-chlorophenyl)-1-(2-furoyl)-5-(trifluoromethyl)pyrrol-3-carbonitril;
4-Bromo-2-(p-chlorophenyl)-1-p-toluoyl-5-(trifluoromethyl)pyrrol-3-carbonitril;
4-Bromo-2-(p-chlorophenyl)-5-(trifluoromethyl)-1-($\alpha$,$\alpha$,$\alpha$-trifluoro-p-toluoyl)pyrrol-3-carbo-nitril;
4-Bromo-2-(p-chlorophenyl)-1-(p-nitrobenzoyl)-5-(trifluoromethyl)pyrrol-3-carbonitril;
Phenyl-3-bromo-5-(p-chlorophenyl)-4-cyano-2-(trifluoromethyl)pyrrol-1-carboxylat;
4-Bromo-1-(p-chlorophenyl)-2-(p-chlorophenyl)-5-(trifluoromethyl)pyrrol-3-carbonitril;
4-Bromo-2-(p-chlorophenyl)-1-(cyclohexylcarbonyl)-5-(trifluoromethyl)pyrrol-3-carbonitril;
4-Bromo-2-(p-chlorophenyl)-1-pivaloyl-5-(trifluoromethyl)pyrrol-3-carbonitril;
1-Acetyl-4,5-dichloro-2-(3,4-dichlorophenyl)-pyrrol-3-carbonitril;
Phenyl-2,3-dibromo-4-cyano-5-($\alpha$,$\alpha$,$\alpha$-trifluoro-p-tolyl)pyrrol-3-carboxylat;
4-Bromo-2-(p-chlorophenyl)-1-(1-naphthoyl)-5-(trifluoromethyl)pyrrol-3-carbonitril;
4-Bromo-2-(p-chlorophenyl)-1-(m-fluorobenzoyl)-5-(trifluoromethyl)pyrrol-3-carbonitril;
4-Bromo-2-(p-chlorophenyl)-1-(3,4-dichlorobenzoyl)-5-(trifluoromethyl)pyrrol-3-carbonitril;
1-Benzoyl-4,5-dichloro-2-(3,4-dichlorophenyl)-pyrrol-3-carbonitril;
4-Bromo-1-(p-tert-butylbenzoyl)-2-(p-chloro-phenyl)-5-(trifluoromethyl)pyrrol-3-carbo-nitril;
1-Benzoyl-4-bromo-2-(p-chlorophenyl)-5-(trifluoromethyl)pyrrol-3-carbonitril; ist.

**6.** Verfahren gemäß Anspruch 5, worin das N-acylierte Arylpyrrol
1-Benzoyl-4-bromo-2-(p-chlorophenyl)-5-(trifluoromethyl)pyrrol-3-carbonitril;
4-Bromo-2-(p-chlorophenyl)-1-methacryloyl-5-(trifluoromethyl)pyrrol-3-carbonitril; oder
4-Bromo-2-(p-chlorophenyl)-1-o-toluoyl-5-(trifluoromethyl)pyrrol-3-carbonitril; ist.

**7.** Verwendung einer Verbindung, hergestellt gemäß Anspruch 1, in einem Verfahren zur Bekämpfung von Insekten, Akariden, Nematoden, Pilzen oder Mollusken, umfassend das Inberührungbringen der besagten Insekten, Akariden, Nematoden, Pilze oder Mollusken, ihre Fortpflanzungsstätten, Nahrung oder Lebensraum mit einer insektizid, akarizid, nematizid, fungizid oder molluskizid wirksamen Menge eines N-acylierten Arylpyrrol.

**8.** Verwendung gemäß Anspruch 7, worin besagtes N-acyliertes Arylpyrrol auf besagte Insekten, Akariden, Nematoden, Pilze oder Mollusken, ihre Fortpflanzungsstätten, Nahrung oder Lebensraum in ausreichender Menge angewendet wird, um eine aktive Bestandteilmenge von 0.1 kg/ha bis 4.0 kg/ha bereitzustellen.

**9.** Verwendung gemäß Anspruch 7, worin besagtes N-acyliertes Arylpyrrol die allgemeine Formel

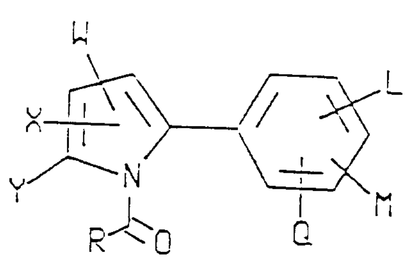

hat und worin R wie in Anspruch 4 definiert ist.

**Revendications**

**Revendications pour les Etats contractants suivants : AT, BE, CH, DE, DK, FR, GB, GR, IT, LI, LU, NL, SE**

**1.** Composé de type arylpyrrole N-acylé représenté par la formule structurale I :

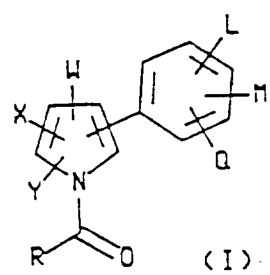

(I)

dans laquelle

X est H, F, Cl, Br, I ou $CF_3$ ;

Y est F, Cl, Br, I ou $CF_3$ ;

W est CN ou $NO_2$ ;

L est H, F, Cl ou Br ;

M et Q sont chacun indépendamment H, un groupe alkyle en $C_1$-$C_3$, alcoxy en $C_1$-$C_3$, alkylthio en $C_1$-$C_3$, alkylsulfinyle en $C_1$-$C_3$, alkylsulfonyle en $C_1$-$C_3$, cyano, F, Cl, Br, I, nitro, $CF_3$, $R_1CF_2Z$, $R_2CO$ ou $NR_3R_4$ ;
et si M et Q sont liés à des atomes de carbone adjacents du noyau phénylique , pris avec les atomes de carbone auxquels ils sont liés , ils peuvent former un cycle dans lequel MQ représente la structure :
-$OCH_2O$-, -$OCF_2O$- ou

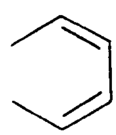

;

Z est $S(O)_n$ ou O ;

$R_1$ est H, F, $CHF_2$, CHFCl ou $CF_3$ ;

$R_2$ est un groupe alkyle en $C_1$-$C_3$, alcoxy en $C_1$-$C_3$ ou $NR_3R_4$ ;

$R_3$ est H ou un groupe alkyle en $C_1$-$C_3$ ;

$R_4$ est H, un groupe alkyle en $C_1$-$C_3$ ou $R_5CO$ ;

$R_5$ est H ou un groupe alkyle en $C_1$-$C_3$ ;

n est le nombre entier 0, 1 ou 2 ; et

R est un groupe alkyle en $C_1$-$C_6$ facultativement substitué par
un à trois atomes d'halogènes,
un groupe hydroxyle,
un groupe cyano,
un ou deux groupes alcoxy en $C_1$-$C_4$ facultativement substitués par un à trois atomes d'halogènes,
un groupe alkylthio en $C_1$-$C_4$,
un groupe phényle facultativement substitué par un à trois atomes d'halogènes, un a trois groupes alkyle en $C_1$-$C_4$ ou un à trois groupes alcoxy en $C_1$-$C_4$,
un groupe phénoxy facultativement substitué par un à trois atomes d'halogènes, un à trois groupes alkyle en $C_1$-$C_4$ ou un à trois groupes alcoxy en $C_1$-$C_4$,
un groupe benzyloxy facultativement substitué sur le noyau phénylique par un a trois atomes d'halogènes, un à trois groupes alkyle en $C_1$-$C_4$ ou un à trois groupes alcoxy en $C_1$-$C_4$,
un groupe (alkyle en $C_1$-$C_6$)carbonyloxy facultativement substitué par un a trois

atomes d'halogènes,

un groupe (alcényle en $C_2$-$C_6$)carbonyloxy facultativement substitué par un a trois atomes d'halogènes,

un groupe phénylcarbonyloxy facultativement substitué par un à trois atomes d'halogènes, un à trois groupes alkyle en $C_1$-$C_4$ ou un à trois groupes alcoxy en $C_1$-$C_4$,

un groupe (alcoxy en $C_1$-$C_6$)carbonyle facultativement substitué par un à trois atomes d'halogènes ou un à trois groupes alcoxy en $C_1$-$C_4$, ou

un groupe benzyloxycarbonyle facultativement substitué sur le noyau phénylique par un à trois atomes d'halogènes, un à trois groupes alkyle en $C_1$-$C_4$ ou un à trois groupes alcoxy en $C_1$-$C_4$,

un groupe alcényle en $C_2$-$C_6$ facultativement substitué par un à trois atomes d'halogènes ou un groupe phényle,

un groupe alcynyle en $C_3$-$C_6$ facultativement substitué par un à trois atomes d'halogènes ou un groupe phényle,

un groupe cycloalkyle en $C_3$-$C_6$,

un groupe phényle facultativement substitué par un a trois atomes d'halogènes, un a trois groupes alkyle en $C_1$-$C_4$, un groupe alkyle en $C_5$-$C_{12}$, un ou deux groupes alcoxy en $C_1$-$C_4$ ou un groupe phénoxy, alkylthio en $C_1$-$C_4$, trialkylsilyle, alkylsulfinyle en $C_1$-$C_4$, alkylsulfonyle en $C_1$-$C_4$, (alcoxy en $C_1$-$C_4$)carbonyle, carboxyle, $CF_3$, CN, $NO_2$, di(alkyle en $C_1$-$C_4$)amino ou alcanoylamino en $C_1$-$C_4$,

un groupe phénoxy facultativement substitué par un à trois atomes d'halogènes, un ou deux groupes alkyle en $C_1$-$C_4$, un ou deux groupes alcoxy en $C_1$-$C_4$, trialkylsilyle, $CF_3$, CN, $NO_2$ ou di(alkyle en $C_1$-$C_4$)amino ou un groupe alcanoylamino en $C_1$-$C_4$,

un groupe 1- ou 2-naphtyle,

un groupe 2-, 3- ou 4-pyridyle facultativement substitué par un a trois atomes d'halogènes,

un cycle hétéroaromatique pentagonal contenant un atome d'oxygène, d'azote ou de soufre, et facultativement substitué par un à trois atomes d'halogènes,

un groupe alcoxy en $C_1$-$C_6$ facultativement substitué par un à trois atomes d'halogènes ; ou

un groupe alcényloxy en $C_2$-$C_6$ facultativement substitué par un à trois atomes d'halogènes.

**2.** Composé selon la revendication 1, dans lequel le composé répond à la formule

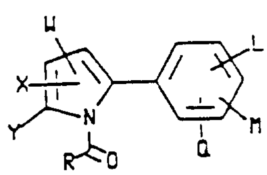

et dans lequel R est

un groupe phényle facultativement substitué par un a trois atomes d'halogènes, un à trois groupes alkyle en $C_1$-$C_4$, un ou deux groupes alcoxy en $C_1$-$C_4$, ou un groupe phénoxy, alkylthio en $C_1$-$C_4$, trialkylsilyle, alkylsulfinyle en $C_1$-$C_4$, alkylsulfonyle en $C_1$-$C_4$, (alcoxy en $C_1$-$C_4$)carbonyle, carboxyle, $CF_3$, CN, $NO_2$, di(alkyle en $C_1$-$C_4$)amino ou alcanoylamino en $C_1$-$C_4$,

un groupe 1-naphtyle ou 2-naphtyle,

un groupe 2-, 3- ou 4-pyridyle facultativement substitué par un à trois atomes d'halogènes, ou

un cycle hétéroaromatique pentagonal contenant un atome d'oxygène, d'azote ou de soufre, et facultativement substitué par un a trois atomes d'halogènes.

**3.** Composé selon la revendication 1, dans lequel ledit arylpyrrole N-acylé est :

le 1-benzoyl-4-bromo-2-(*p*-chlorophényl)-5-(trifluorométhyl)pyrrole-3-carbonitrile;

le 4-bromo-2-(*p*-chlorophényl)-1-méthacryloyl-5-(trifluorométhyl)pyrrole-3-carbonitrile ;

le 4-bromo-2-(*p*-chlorophényl)-1-*o*-toluoyl-5-(trifluorométhyl)pyrrole-3-carbonitrile ;

le 4-bromo-1-(*m*-chlorobenzoyl)-1-(*p*-chlorophényl)-5-(trifluorométhyl)pyrrole-3-carbonitrile;

le 4-bromo-2-(*p*-chlorophényl)-1-(2-furoyl)-5-(trifluorométhyl)pyrrole-3-carbonitrile ;
le 4-bromo-2-(*p*-chlorophényl)-1-*p*-toluoyl-5-(trifluorométhyl)pyrrole-3-carbonitrile ;
le 4-bromo-2-(*p*-chlorophényl)-5-(trifluorométhyl)-1-(α,α,α-trifluoro-*p*-toluoyl)pyrrole-3-carbonitrile ;
le 4-bromo-2-(*p*-chlorophényl)-1-(*p*-nitrobenzoyl)-5-(trifluorométhyl)pyrrole-3-carbonitrile ;
le 3-bromo-5-(*p*-chlorophényl)-4-cyano-2-(trifluorométhyl)-pyrrole-1-carboxylate de phényle ;
le 4-bromo-1-(*p*-chlorobenzoyl)-2-(*p*-chlorophényl)-5-(trifluorométhyl)pyrrole-3-carbonitrile;
le 4-bromo-2-(*p*-chlorophényl)-1-(cyclohexylcarbonyl)-5-(trifluorométhyl)pyrrole-3-carbonitrile ;
le 1-benzoyl-4-bromo-2-chloro-5-(*p*-chlorophényl)pyrrole-3-carbonitrile ;
le 4-bromo-2-(*p*-chlorophényl)-1-pivaloyl-5-(trifluorométhyl)pyrrole-3-carbonitrile ;
le 1-acétyl-4,5-dichloro-2-(3,4-dichlorophényl)pyrrole-3-carbonitrile ;
le 2,3-dibromo-4-cyano-5-(α,α,α-trifluoro-*p*-tolyl)pyrrole-1-carboxylate de phényle ;
le 4-bromo-2-(*p*-chlorophényl)-1-(1-naphtoyl)-5-(trifluorométhyl)pyrrole-3-carbonitrile ;
le 4-bromo-2-(*p*-chlorophényl)-1-(*m*-fluorobenzoyl)-5-(trifluorométhyl)pyrrole-3-carbonitrile;
le 4-bromo-2-(*p*-chlorophényl)-1-(3,4-dichlorobenzoyl)-5-(trifluorométhyl)pyrrole-3-carbonitrile ;
le 4-bromo-1-(*p*-*tert*-butylbenzoyl)-2-(*p*-chlorophényl)-5-(trifluorométhyl)pyrrole-3-carbonitrile; ou
le l-benzoyl-4-bromo-2-(*p*-bromophényl)-5-(trifluorométhyl)pyrrole-3-carbonitrile.

**4.** Composé selon la revendication 3, dans lequel l'arylpyrrole N-acylé est le 1-benzoyl-4-bromo-2-(*p*-chlorophényl)-5-(trifluorométhyl)pyrrole-3-carbonitrile; le 4-bromo-2-(*p*-chlorophényl)-1-méthacroylyl)-5-(trifluorométhyl)pyrrole-3-carbonitrile ; ou le 4-bromo-2-(*p*-chlorophényl)-1-*o*-toluoyl-5-(trifluorométhyl)-pyrrole-3-carbonitrile.

**5.** Composé selon la revendication 1 à utiliser dans une méthode de lutte contre des insectes, acariens, nématodes, champignons ou mollusques qui comprend la mise en contact lesdits insectes, acariens, nématodes, champignons ou mollusques,de leurs lieux de reproduction, de leur source de nourriture ou de leur habitat avec une quantité insecticide, acaricide, nématicide, fongicide ou molluscicide efficace d'un arylpyrrole N-acylé.

**6.** Méthode selon la revendication 5, dans laquelle ledit arylpyrrole N-acylé de formule I est appliqué auxdits insectes, acariens, nématodes, champignons ou mollusques nuisibles, à leurs lieux de reproduction, à leur source de nourriture ou à leur habitat en une quantité suffisante pour fournir une dose de 0,1 kg/ha à environ 4,0 kg/ha d'ingrédient actif.

**7.** Méthode selon la revendication 5, dans laquelle ledit arylpyrrole N-acylé répond à la formule

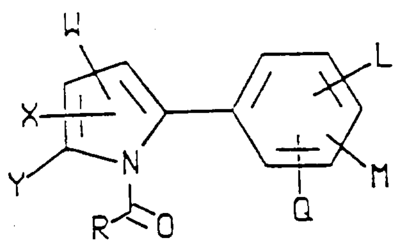

et dans laquelle R est tel que défini dans la revendication 2.

**8.** Procédé de préparation d'arylpyrroles N-acylés tels que définis dans la revendication 1, qui comprend la réaction d'un arylpyrrole ayant la structure :

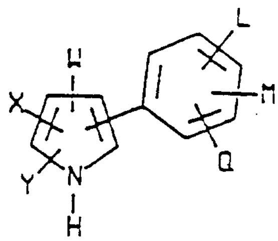

où L, M, Q, W, X et Y sont tels que décrits dans la revendication 1, avec un excès d'un hydrure de métal alcalin ou *t*-butylate de métal alcalin, en présence d'un solvant organique inerte anhydre pour former un premier mélange, l'addition du chlorure de carbonyle convenablement substitué ayant la structure : RCOCl, où R est tel que décrit dans la revendication 1, pour former un second mélange, le chauffage de ce second mélange pour former ledit arylpyrrole N-acylé.

**9.** Procédé selon la revendication 8, qui comprend de plus le chauffage dudit premier mélange à la température de reflux, puis le refroidissement dudit premier mélange jusqu'à une température comprise entre environ 20°C et 30°C, et dans lequel ledit second mélange est chauffé à la température de reflux.

**10.** Procédé selon la revendication 8, dans lequel l'hydrure de métal alcalin est l'hydrure de sodium, le solvant organique est le tétrahydrofuranne, le diméthylformamide ou le diméthylsulfoxyde et la réaction de l'arylpyrrole avec l'hydrure de sodium est conduite sous une atmosphère protectrice d'azote.

**Revendications pour l'Etat contractant suivant : ES**

**1.** Procédé de préparation d'un arylpyrrole N-acylé représenté par la formule structurale I :

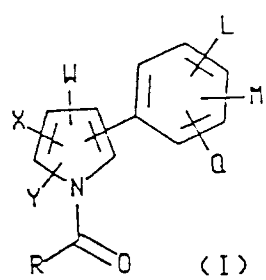
(I)

dans laquelle
- X est H, F, Cl, Br, I ou $CF_3$ ;
- Y est F, Cl, Br, I ou $CF_3$ ;
- W est CN ou $NO_2$ ;
- L est H, F, Cl ou Br ;
- M et Q sont chacun indépendamment H, un groupe alkyle en $C_1$-$C_3$, alcoxy en $C_1$-$C_3$, alkylthio en $C_1$-$C_3$, alkylsulfinyle en $C_1$-$C_3$, alkylsulfonyle en $C_1$-$C_3$, cyano, F, Cl, Br, I, nitro, $CF_3$, $R_1CF_2Z$, $R_2CO$ ou $NR_3R_4$ ;
  et si M et Q sont liés à des atomes de carbone adjacents du noyau phénylique, pris avec les atomes de carbone auxquels ils sont liés, ils peuvent former un cycle dans lequel MQ représente la structure :
  $-OCH_2O-$, $-OCF_2O-$ ou

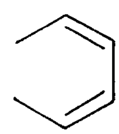
;

Z est $S(O)_n$ ou O ;
$R_1$ est H, F, $CHF_2$, CHFCl ou $CF_3$ ;
$R_2$ est un groupe alkyle en $C_1$-$C_3$, alcoxy en $C_1$-$C_3$ ou $NR_3R_4$ ;
$R_3$ est H ou un groupe alkyle en $C_1$-$C_3$ ;
$R_4$ est H, un groupe alkyle en $C_1$-$C_3$ ou $R_5CO$ ;
$R_5$ est H ou un groupe alkyle en $C_1$-$C_3$ ;
n est le nombre entier 0, 1 ou 2 ; et
R est un groupe alkyle en $C_1$-$C_6$ facultativement substitué par
un à trois atomes d'halogènes,
un groupe hydroxyle,
un groupe cyano,
un ou deux groupes alcoxy en $C_1$-$C_4$ facultativement substitués par un à trois atomes d'halogènes,
un groupe alkylthio en $C_1$-$C_4$,
un groupe phényle facultativement substitué par un à trois atomes d'halogènes, un à trois groupes alkyle en $C_1$-$C_4$ ou un a trois groupes alcoxy en $C_1$-$C_4$,
un groupe phénoxy facultativement substitué par un à trois atomes d'halogènes, un à trois groupes alkyle en $C_1$-$C_4$ ou un à trois groupes alcoxy en $C_1$-$C_4$,
un groupe benzyloxy facultativement substitué sur le noyau phénylique par un à trois atomes d'halogènes, un à trois groupes alkyle en $C_1$-$C_4$ ou un à trois groupes alcoxy en $C_1$-$C_4$,
un groupe (alkyle en $C_1$-$C_6$)carbonyloxy facultativement substitué par un à trois atomes d'halogènes,
un groupe (alcényle en $C_2$-$C_6$)carbonyloxy facultativement substitué par un à trois atomes d'halogènes,
un groupe phénylcarbonyloxy facultativement substitué par un à trois atomes d'halogènes, un à trois groupes alkyle en $C_1$-$C_4$ ou un à trois groupes alcoxy en $C_1$-$C_4$,
un groupe (alcoxy en $C_1$-$C_6$)carbonyle facultativement substitué par un à trois atomes d'halogènes ou un à trois groupes alcoxy en $C_1$-$C_4$, ou
un groupe benzyloxycarbonyle facultativement substitué sur le noyau phénylique par un à trois atomes d'halogènes, un a trois groupes alkyle en $C_1$-$C_4$ ou un a trois groupes alcoxy en $C_1$-$C_4$,
un groupe alcényle en $C_2$-$C_6$ facultativement substitué par un a trois atomes d'halogènes ou un groupe phényle,
un groupe alcynyle en $C_3$-$C_6$ facultativement substitué par un a trois atomes d'halogènes ou un groupe phényle,
un groupe cycloalkyle en $C_3$-$C_6$,
un groupe phényle facultativement substitué par un à trois atomes d'halogènes, un a trois groupes alkyle en $C_1$-$C_4$, un groupe alkyle en $C_5$-$C_{12}$, un ou deux groupes alcoxy en $C_1$-$C_4$ ou un groupe phénoxy, alkylthio en $C_1$-$C_4$, trialkylsilyle, alkylsulfinyle en $C_1$-$C_4$, alkylsulfonyle en $C_1$-$C_4$, (alcoxy en $C_1$-$C_4$)carbonyle, carboxyle, $CF_3$, CN, $NO_2$, di(alkyle en $C_1$-$C_4$)amino ou alcanoylamino en $C_1$-$C_4$,
un groupe phénoxy facultativement substitué par un a trois atomes d'halogènes, un ou deux groupes alkyle en $C_1$-$C_4$, un ou deux groupes alcoxy en $C_1$-$C_4$, trialkylsilyle, $CF_3$, CN, $NO_2$ ou di(alkyle en $C_1$-$C_4$)amino ou un groupe alcanoylamino en $C_1$-$C_4$,
un groupe 1- ou 2-naphtyle,
un groupe 2-, 3- ou 4-pyridyle facultativement substitué par un à trois atomes d'halogènes,
un cycle hétéroaromatique pentagonal contenant un atome d'oxygène, d'azote ou de soufre, et facultativement substitué par un à trois atomes d'halogènes,
un groupe alcoxy en $C_1$-$C_6$ facultativement substitué par un à trois atomes d'halogènes ; ou
un groupe alcényloxy en $C_2$-$C_6$ facultativement substitué par un à trois atomes d'halogènes,

qui comprend la réaction d'un arylpyrrole ayant la structure :

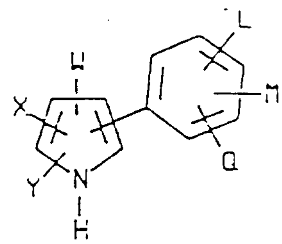

où L, M, Q, W, X et Y sont tels que décrits ci-dessus, avec un excès d'un hydrure de métal alcalin ou *t*-butylate de métal alcalin, en présence d'un solvant organique inerte anhydre pour former un premier mélange, l'addition du chlorure de carbonyle convenablement substitué ayant la structure : RCOCl, où R est tel que décrit ci-dessus, pour former un second mélange, le chauffage de ce second mélange pour former ledit arylpyrrole N-acylé.

**2.** Procédé selon la revendication 1, qui comprend de plus le chauffage dudit premier mélange à la température de reflux, puis le refroidissement dudit premier mélange jusqu'à une température comprise entre environ 20 °C et 30 °C, et dans lequel ledit second mélange est chauffé à la température de reflux.

**3.** Procédé selon la revendication 1, dans lequel l'hydrure de métal alcalin est l'hydrure de sodium, le solvant organique est le tétrahydrofuranne, le diméthylformamide ou le diméthylsulfoxyde et la réaction de l'arylpyrrole avec l'hydrure de sodium est conduite sous une atmosphère protectrice d'azote.

**4.** Procédé selon la revendication 1, dans lequel le composé répond à la formule

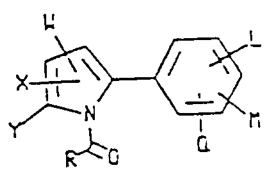

et dans lequel R est

un groupe phényle facultativement substitué par un à trois atomes d'halogènes, un a trois groupes alkyle en $C_1$-$C_4$, un ou deux groupes alcoxy en $C_1$-$C_4$, ou un groupe phénoxy, alkylthio en $C_1$-$C_4$, trialkylsilyle, alkylsulfinyle en $C_1$-$C_4$, alkylsulfonyle en $C_1$-$C_4$, (alcoxy en $C_1$-$C_4$)carbonyle, carboxyle, $CF_3$, CN, $NO_2$, di(alkyle en $C_1$-$C_4$)amino ou alcanoylamino en $C_1$-$C_4$,

un groupe 1-naphtyle ou 2-naphtyle,

un groupe 2-, 3- ou 4-pyridyle facultativement substitué par un à trois atomes d'halogènes, ou

un cycle hétéroaromatique pentagonal contenant un atome d'oxygène, d'azote ou de soufre, et facultativement substitué par un à trois atomes d'halogènes.

**5.** Procédé selon la revendication 1, dans lequel l'arylpyrrole N-acylé est :

le 1-benzoyl-4-bromo-2-(*p*-chlorophényl)-5-(trifluorométhyl)pyrrole-3-carbonitrile;
le 4-bromo-2-(*p*-chlorophényl)-1-méthacroylyl-5-(trifluorométhyl)pyrrole-3-carbonitrile ;
le 4-bromo-2-(*p*-chlorophényl)-1-*o*-toluoyl-5-(trifluorométhyl)pyrrole-3-carbonitrile ;
le 4-bromo-1-(*m*-chlorobenzoyl)-1-(*p*-chlorophényl)-5-(trifluorométhyl)pyrrole-3-carbonitrile;
le 4-bromo-2-(*p*-chlorophényl)-1-(2-furoyl)-5-(trifluorométhyl)pyrrole-3-carbonitrile ;
le 4-bromo-2-(*p*-chlorophényl)-1-*p*-toluoyl-5-(trifluorométhyl)pyrrole-3-carbonitrile ;
le 4-bromo-2-(*p*-chlorophényl)-5-(trifluorométhyl)-1-($\alpha$,$\alpha$,$\alpha$-trifluoro-*p*−toluoyl)pyrrole-3-carbonitrile ;
le 4-bromo-2-(*p*-chlorophényl)-1-(*p*-nitrobenzoyl)-5-(trifluorométhyl)pyrrole-3-carbonitrile ;
le 3-bromo-5-(*p*-chlorophényl)-4-cyano-2-(trifluorométhyl)pyrrole-1-carboxylate de phényle ;
le 4-bromo-1-(*p*-chlorobenzoyl)-2-(*p*-chlorophényl)-5-(trifluorométhyl)pyrrole-3-carbonitrile;
le 4-bromo-2-(*p*-chlorophényl)-1-(cyclohexylcarbonyl)-5-(trifluorométhyl)pyrrole-3-carbonitrile ;
le 1-benzoyl-4-bromo-2-chloro-5-(*p*-chlorophényl)pyrrole-3-carbonitrile ;

le 4-bromo-2-(*p*-chlorophényl)-1-pivaloyl-5-(trifluorométhyl)pyrrole-3-carbonitrile ;
le 1-acétyl-4,5-dichloro-2-(3,4-dichlorophényl)pyrrole-3-carbonitrile ;
le 2,3-dibromo-4-cyano-5-(α,α,α-trifluoro-*p*-tolyl)pyrrole-1-carboxylate de phényle ;
le 4-bromo-2-(*p*-chlorophényl)-1-(1-naphtoyl)-5-(trifluorométhyl)pyrrole-3-carbonitrile ;
le 4-bromo-2-(*p*-chlorophényl)-1-(*m*-fluorobenzoyl)-5-(trifluorométhyl)pyrrole-3-carbonitrile;
le 4-bromo-2-(*p*-chlorophényl)-1-(3,4-dichlorobenzoyl)-5-(trifluorométhyl)pyrrole-3-carbonitrile ;
le 4-bromo-1-(*p-tert*-butylbenzoyl)-2-(*p*-chlorophényl)-5-(trifluorométhyl)pyrrole-3-carbonitrile; ou
le l-benzoyl-4-bromo-2-(*p*-bromophényl)-5-(trifluorométhyl)pyrrole-3-carbonitrile.

**6.** Procédé selon la revendication 5, dans lequel l'arylpyrrole N-acylé est le 1-benzoyl-4-bromo-2-(*p*-chlorophényl)-5-(trifluorométhyl)pyrrole-3-carbonitrile; le 4-bromo-2-(*p*-chlorophényl)1-méthacroylyl)-5-(trifluorométhyl)pyrrole-3-carbonitrile ; ou le 4-bromo-2-(*p*-chlorophényl)-1-*o*-toluoyl-5-(trifluorométhyl)-pyrrole-3-carbonitrile.

**7.** Utilisation d'un composé préparé selon la revendication 1 dans une méthode de lutte contre des insectes, acariens, nématodes, champignons ou mollusques qui comprend la mise en contact lesdits insectes, acariens, nématodes, champignons ou mollusques, de leurs lieux de reproduction,de leur source de nourriture ou de leur habitat avec une quantité insecticide, acaricide, nématicide, fongicide ou molluscicide efficace d'un arylpyrrole N-acylé.

**8.** Utilisation selon la revendication 7, dans laquelle ledit arylpyrrole N-acylé de formule I est appliqué auxdits insectes acariens, nématodes, champignons ou mollusques nuisibles, à leurs lieux de reproduction, à leur source de nourriture ou à leur habitat en une quantité suffisante pour fournir une dose de 0,1 kg/ha à environ 4,0 kg/ha d'ingrédient actif.

**9.** Utilisation selon la revendication 7, dans laquelle ledit arylpyrrole N-acylé répond à la formule

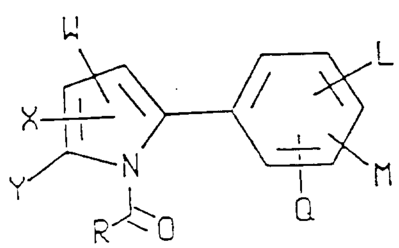

et où R est tel que défini dans la revendication 4.